# EUROPÄISCHE PATENTSCHRIFT

(11) **EP 2 303 948 B1**
(45) Veröffentlichungstag und Bekanntmachung des Hinweises auf die Patenterteilung: **12.09.2018**
(21) Anmeldenummer: 09776935.0
(22) Anmeldetag: 03.07.2009
(51) Int. Cl.: C08G 77/06, A61K 9/00, A61K 47/02, A61L 15/64, C04B 35/622

(54) **VERFAHREN ZUR HERSTELLUNG EINES KIESELSOL-MATERIALS MIT MINDESTENS EINEM THERAPEUTISCH AKTIVEN WIRKSTOFF ZUR HERSTELLUNG VON BIOLOGISCH DEGRADIERBAREN UND/ODER RESORBIERBAREN KIESELGEL-MATERIALEN FÜR DIE HUMANMEDIZIN UND/ODER MEDIZINTECHNIK**
PROCESS FOR THE MANUFACTURE OF SILICA SOL MATERIAL HAVING AT LEAST ONE THERAPEUTICALLY ACTIVE SUBSTANCE FOR PRODUCING BIOLOGICALLY DEGRADABLE AND/OR RESORBABLE SILICA GEL MATERIALS FOR HUMAN MEDICINE AND/OR MEDICAL TECHNOLOGY
PRÉPARATION D'UN MATÉRIAU À BASE DE SOL DE SILICE PRÉSENTANT AU MOINS UNE SUBSTANCE THÉRAPEUTIQUEMENT ACTIVE POUR LA PRÉPARATION DE MATÉRIAUX À BASE DE GEL DE SILICE BIODÉGRADABLES ET/OU BIORÉSORBABLES POUR LA MÉDECINE HUMAINE ET/OU LA TECHNIQUE MÉDICALE

(30) Priorität: 16.07.2008 DE 102008033327
(43) Veröffentlichungstag der Anmeldung: 06.04.2011
(73) Patentinhaber: Bayer Intellectual Property GmbH, 40789 Monheim (DE)
(72) Erfinder: BAECKER, Iwer, 40219 Düsseldorf (DE); ROTHENBURGER GLAUBITT, Miranda, 97276 Margetshöchheim (DE); PROBST, Jörn, 97082 Würzburg (DE); EGGER, Holger, 51067 Köln (DE)
(74) Vertreter: Kutzenberger Wolff & Partner
(86) Internationale Anmeldenummer: PCT/EP2009/004806
(87) Internationale Veröffentlichungsnummer: WO 2010/006708

(56) Entgegenhaltungen:
- WO-A1-00/47236
- WO-A1-97/45367
- DE-T2- 60 035 672
- US-B1- 7 118 921
- GILL I; BALLESTEROS A: "Bioencapsulation within synthetic polymers (Part 1): sol-gel encapsulated biologicals" TRENDS IN BIOTECHNOLOGY, ELSEVIER PUBLICATIONS, CAMBRIDGE, GB, Bd. 18, Nr. 7, 1. Juli 2000 (2000-07-01), Seiten 282-296, XP004908534 ISSN: 0167-7799

## Beschreibung

Die Erfindung betrifft ein Verfahren zur Herstellung eines Kieselsol-Materials mit mindestens einem therapeutisch aktiven Wirkstoff zur Herstellung von biologisch degradierbaren und/oder resorbierbaren Kieselgel-Materialien für die Humanmedizin und/oder Medizintechnik.

Es gibt vielfältige Bestrebungen biologisch degradierbare und/oder biologisch resorbierbare Materialien für verschiedene Anwendungen in der Humanmedizin und der Medizintechnik zu entwickeln. In diesen Bereichen werden zudem stetig steigende Anforderungen, insbesondere an die biologische Verträglichkeit, biologische Wirksamkeit und die toxikologischen Eigenschaften der Materialien, gestellt.

Resorbierbare Kieselgele sind im Stand der Technik bekannt. In der DE 196 09 551 C1 sind biologisch degradierbare, biologisch resorbierbare Faserstrukturen beschrieben. Diese Fasern können in einem Sol-Gel-Prozess dadurch erhalten werden, dass man aus einer Spinnmasse Fäden zieht und diese gegebenenfalls trocknet. Die Spinnmasse enthält eine oder mehrere partiell oder vollständig hydrolytisch kondensierte Verbindungen des Siliziums, die sich durch hydrolytische Kondensation ableiten von Monomeren der allgemeinen Formel SiX₄. Diese Fasern haben den Nachteil, dass sie bei einer Degradation direkt nach dem Spinnprozess noch keine optimalen Ergebnisse in Cytotoxizitätstests zeigen und teilweise als cytotoxisch bewertet werden müssen. Eine solche Toxizität ist gerade im Einsatz in der Humanmedizin oder Medizintechnik, zum Beispiel im Bereich der Wundheilung, in der Regel nicht erwünscht. Das Verfahren zur Herstellung der Fasern nach der DE 196 09 551 C1 hat darüber hinaus den Nachteil, dass die resultierende Mischung nach dem Entfernen des Lösungsmittels im Hydrolyse-Kondensationsschritt schon eine mehrphasige Mischung ist und zum Entfernen des entstandenen Feststoffs gezwungenermaßen einer Filtration unterworfen werden muss. Darüber hinaus geht durch die Bildung der festen Phase und durch den zwingenden Filtrationsschritt ein großer Anteil des spinnbaren Sols verloren. Nach dem Verfahren der DE 196 09 551 C1 kann auch während der Reifung die Bildung eines nicht unbeträchtlichen Anteils einer festen Phase, insbesondere eine Gelbildung, nicht sicher unterdrückt werden. Dies reduziert nochmals den Anteil an spinnbarer Sol-Masse.

Unabhängig davon konnte gezeigt werden, dass die Fasern und Vliesen verbesserte Wundheilungseigenschaften aufweisen. Darüber hinaus sind die Fasern und Vliesen besonders für den Einsatz als Zellstützstrukturen geeignet.

DE 600 35 672 T2 bezieht sich auf ein Verfahren zur Einstellung der biologischen Abbaubarkeit von Silicafasern, umfassend Spinnen der Fasern aus einem Silicasol, wobei die Viskosität des Sols, aus dem die Faser gesponnen wird, ausgewählt wird, sowie auf kontrollierbar biologisch abbaubare Fasern als Abgabevorrichtungen für eine anhaltende und/oder kontrollierte Freisetzung für biologisch aktive Agenzien.

Aufgabe der vorliegenden Erfindung ist es, ein neues Kieselsol-Material mit mindestens einem therapeutisch aktiven Wirkstoff für die Herstellung von biologisch degradierbaren und/oder biologisch resorbierbaren Kieselgel-Materialien zur Verfügung zu stellen. Außerdem ist es Aufgabe der vorliegenden Erfindung biologisch degradierbare und/oder biologisch resorbierbare Kieselgel-Materialien mit mindestens einem therapeutisch aktiven Wirkstoff bereitzustellen, die verbesserte Cytotoxizitäts- und/oder Wundheilungseigenschaften aufweisen. Eine weitere Aufgabe kann darin gesehen werden, verbesserte Zellstützstrukturen beispielsweise zur *in-vitro* Herstellung von Hautimplantaten, Knorpel und Knochen zur Verfügung zu stellen.

Die Aufgabe wird durch ein Verfahren gemäß Anspruch 1 gelöst.

Erfindungsgemäß wird Tetraethoxysilan (TEOS) als Si-Verbindung in die erfindungsgemäße Hydrolyse-Kondensations-Reaktion eingesetzt. Als wasserlösliches Lösungsmittel kann bevorzugt Ethanol bzw. ein Wasser/Ethanol Gemisch eingesetzt werden. Die Si-Verbindung kann zum Ethanol in einem Verhältnis von ≥ 1 eingesetzt werden.

Der anfängliche pH von 0 bis ≤ 7. vorzugsweise von 2 bis 5, wird in einer bevorzugten Ausführungsform der Erfindung mit und salpetersaurem Wasser eingestellt. Andere saure Gemische und/oder Lösungen, die lokal NO oder NO₂ erzeugen können, sind jedoch für die Ausführung der vorliegenden Erfindung auch geeignet. Dies können z.B. saure Gemische und/oder Lösungen sein, die in physiologischer Umgebung mit molekularem Sauerstoff enzymatisch (mittels einer Nitroxid-Synthase, NOS) Stickstoffmonoxid (NO) erzeugen, das wiederum vom Körper schnell zu NO₂ umgesetzt wird oder es können auch organische Nitrate bzw. Nitratester (so genannte NO-Donatoren) sein z.B. Ethylnitrat, die NO mit Hilfe einer organischen Nitratreduktase bilden. Für diese enzymatische Freisetzung von NO werden Thiolgruppen (Cystein) benötigt.

Neben der verdünnten Salpetersäure ist deshalb erfindungsgemäß auch eine wässrige oder alkoholische (besonders bevorzugt: eine wässrig verdünnte ethanolische) Lösung einer physiologisch verträglichen Säure (z.B. Zitronen-. Bernstein-, Wein-, Essig- oder Ascorbinsäure) und mindestens einer essentiellen (z.B. L-Arginin, besonders bevorzugt; L-Valin, L-Leucin, L-Isoleucin, L-Phenylalanin, L-Thyroxin, L-Methionin, L-Lycin oder L-Tryptophan) oder nichtessentiellen Aminosäure (z.B. L-Glutamin, L-Glutaminsäure, L-Asparagin, L-Asparaginsäure, L-Cystein, L-Glycin, L-Alanin. L-Prolin, L-Histidin, L-Tyrosin) als Substrat der NOS geeignet, den pH auf den gewünschten Wert im schwach bis mittelstark sauren Bereich einzustellen.

In einer bevorzugten Ausführungsform wird die Hydrolyse-Kondensations-Reaktion mit einer Si-Verbindung und salpetersaurem Wasser in einem molaren Verhältnis zwischen 1:1,7 bis 1:1,9, besonders bevorzugt in einem Verhältnis zwischen 1:1,7 und 1:1,8 durchgeführt. Das salpetersaure Wasser kann als 0,01 N HNO₃ eingesetzt werden.

Die Hydrolyse-Kondensation wird über einen Zeitraum von mindestens 16 h, bevorzugt von mindestens 18 h, bei einer Temperatur von 0 °C bis 80 °C, vorzugsweise von 0 °C bis 78 °C besonders bevorzugt bei 20-60 °C, noch bevorzugter bei etwa 20 °C bis etwa 50 °C und zum Beispiel - bei der Verwendung der erfindungsgemäßen Materialien für die Wundbehandlung - bei Raumtemperatur (etwa 20 bis etwa 25 °C) oder bei etwa 37 °C durchgeführt.

In einer bevorzugten Ausführungsform der vorliegenden Erfindung kann die Hydrolyse über einen Zeitraum bevorzugt mindestens 18 Stunden bis zu 4 Wochen durchgeführt werden. Bevorzugt wird die Hydrolysezeit auf 24 h bis zu 18 Tagen, besonders bevorzugt auf 3 bis 8 Tage bemessen. Es wurde überraschend festgestellt, dass bei einer verlängerten Hydrolyse-Kondensationszeit gegenüber den bisher üblichen Zeiten von wenigen Stunden bei Raumtemperatur, nach dem Entfernen des Lösungsmittels in Schritt b) eine homogene einphasige Lösung erhalten werden kann, die vor der Reifung in Schritt d) keiner Filtration mehr bedarf.

Die erste Hydrolyse-Kondensationsreaktion wird vorzugsweise diskontinuierlich in einem Rührwerksbehälter bwz. einem Einhaltsrundkolben mit Rührstab durchgeführt. TEOS und das Solvens (z.B. Ethanol) werden vorzugsweise vorgelegt. Anschließend erfolgt die zügige Zugabe der Säure, vorzugsweise in Form von 0,01 N HNO₃ (z.B. 0.01 Mol HNO₃ pro Mol TEOS). Aufgrund der Säure-Stärke in dem Reaktionsgemisch läuft die erste Hydrolyse-Kondensationsreaktion schnell ab, und der Inhalt des Behälters erwärmt sich um etwa 40°C, ehe die Temperatur noch während der Reaktionszeit (also in Schritt (a)) zu sinken beginnt (in Folge einer natürlichen Abkühlung auf die Umgebungstemperatur, bzw. Heizmitteltemperatur).

Das Entfernen des wasserlöslichen Lösungsmittels (z.B. Ethanol, Wasser) in Schritt (b) wird in einer bevorzugten Ausführungsform der Erfindung in einem geschlossenen Apparat, in dem eine Durchmischung möglich ist (vorzugsweise Rotationsverdampfer und/oder Rührkessel) bei gleichzeitiger Entfernung des Lösungsmittels (Wasser, Ethanol) durch Eindampfen bei einem Druck von 1 bis 1013 mbar, bevorzugt bei einem Druck von < 600 mbar, optional mit kontinuierlicher Zufuhr eines chemisch inerten Schleppgases zur Partialdruckerniedrigung der verdampfenden Komponenten von 1 - 8 m³/h (vorzugsweise bei 2,5 bis 4,5 m³/h), einer Reaktionstemperatur von 30 °C bis 90 °C, vorzugsweise 60-75 °C, noch bevorzugter bei 60-70 °C und vorzugsweise unter schonender Durchmischung des Reaktionssystems bis 80 U/min (vorzugsweise bei 20 U/min bis 60 U/min) bis zu einer Viskosität des Gemisches auf 0,5 bis 30 Pa·s bei einer Scherrate von 10 s⁻¹ bei 4 °C, vorzugsweise 0,5 bis 2 Pa·s bei einer Scherrate von 10 s⁻¹ bei 4 °C, besonders bevorzugt ca. 1 Pa · s (Messung bei 4 °C, Scherrate 10 s⁻¹), durchgeführt.

"Schleppgasstrom" bezeichnet erfindungsgemäß einen Gasstrom, der dem Gasvolumen über der Flüssigphase des Reaktionssystems zugeführt wird. Zur Wahrung der isobaren Verhältnisse im Reaktionsgefäß muss dadurch ein gasförmiger Volumenstrom abgeführt werden, der sowohl aus dem "Schleppgas" als auch aus der / den zu verdampfenden Komponente(n) besteht. Die resultierende Partialdruckerniedrigung, also die Verringerung des Anteils der zu verdampfenden Komponente bzw. des Komponentengemisches im Gasraum, erhöht die treibende Kraft zur Verdampfung des Lösungsmittels an der Flüssigkeitsoberfläche.

In einer besonders bevorzugten Ausführungsform wird der "Schleppgasstrom" mittels eines geeignet im Gasraum des Apparates angeordneten Gasverteilers derart verteilt, dass ein ausreichender Schleppgasaustausch knapp oberhalb der Flüssigkeitsoberfläche gewährleistet wird, ohne jedoch die Flüssigkeitsoberfläche direkt konvektiv anzuströmen. Letzteres kann im Extremfall zu einer lokalen Vergelung führen, was unerwünscht ist. Gasverteiler mittels der diese Ausführungsform umgesetzt werden kann, sind dem Fachmann bekannt.

Durch die fortschreitende Reaktion/Polymerisation (am Viskositätsanstieg erkennbar) verschiebt sich das Phasengleichgewicht, so dass der entsprechende Gleichgewichtsdruck des Lösungsmittels in der Dampfphase immer niedriger wird. Sinkt der Gleichgewichtsdruck auf den Gesamtdruck in der Gasphase ab, hört die Verdampfung auf.

Um weiter Lösungsmittel zu verdampfen, muss optimalerweise daher der Druck abgesenkt, der Schleppgasstrom variable angepasst und/oder die Temperatur erhöht werden.

In einer bevorzugten Ausführungsform der vorliegenden Erfindung wird zumindest einer der Verfahrensparameter Druck, Schleppgasstrom und/oder Temperatur zeitlich variabel angepasst wird.

In einer bevorzugten Ausführungsform der Erfindung erfolgt das Eindampfen in Schritt b) bei einer konstanten Temperatur und zeitlich variablem Druck. In einer bevorzugten Ausführungsform der Erfindung wird als chemisch inerter Schleppgasstrom Stickstoff und/oder Luft zur Partialdruckerniedrigung eingesetzt.

In einer bevorzugten Ausführungsform der Erfindung wird das wasserlösliche Lösungsmittel mittels einer Kombination aus Vakuum und Schleppgasstrom entfernt. Der Gesamtdruck und Schleppgasstrom kann bei dieser Ausführungsform der Erfindung unabhängig voneinander konstant oder zeitlich variabel angepasst werden. In dieser Ausführungsform der Erfindung wird idealerweise zumindest einer der Verfahrensparameter Druck, Schleppgasstrom und/oder Temperatur zeitlich variabel angepasst. Dadurch wird es möglich z. B. integral eine bestimmte Reaktionszeit bei einem gewünschten Eindampfgrad zu erreichen und/oder die Eindampfrate an die Reaktionskinetik anzupassen.

In einer bevorzugten Ausführungsform der Erfindung wird das Eindampfen in Schritt b) bei einer konstanten Temperatur und zeitlich variablem Druck erfolgen, wobei der Druck bis zum Ende der zweiten HKR, ausgehend von Normaldruck bzw. leichtem Unterdruck, auf < 600 mbar, vorzugsweise < 500 mbar, besonders bevorzugt < 100 mbar abgesenkt wird.

Bei der Kombinationsfahrweise (Vakkum mit Schleppgasstrom) ist ein konstanter oder variabler Unterdruck von < 600 mbar bevorzugt.

Temperaturen oberhalb von 60 °C sind besonders bevorzugt, um bei der sonst im Restlösungsmittel deutlich ansteigenden Konzentration der HNO₃ eine reduktive Umsetzung der HNO₃ zu NO zu begünstigen. Dieses sehr leichtflüchtige Gas (Normalsiedepunkt etwa -150 °C) wird nach Entweichen aus der flüssigen Phase bei Luftkontakt zum leicht siedenden NO₂ (SP etwa 21 °C) oxidiert, das mit dem Abluft- bzw. Gasstrom aus dem System entfernt wird. Auf diesem Weg wird die Säurekonzentration im erfindungsgemäßen Materials beschränkt bzw. reduziert. Alternativ kann die Säurestärke jedoch auch in einem der nachfolgenden Schritte, z. B. durch Ablüften des festen Körpers, z.B. als Faservlies, reduziert werden.

Wird aber das System organische Säure/Arginin an Stelle von Salpetersäure verwendet, erfolgt die Erhöhung des pH bzw. die Verringerung der Säure-Stärke, falls erwünscht, z.B. mittels Tris-Lösungen (soweit sich die Säure, z.B Essigsäure, nicht austreiben läßt) kurz vor der Applikation durch Spülen in einer wässrigen Tris-Lösung.

Überraschenderweise wurde im Vergleich zu der DE 196 09 551 C1 entdeckt, dass durch eine schonende Durchmischung des Reaktionssystems bei 20 U/min bis 80 U/min, die Ausbildung eines Konzentrationsgradienten über der Höhe des Ansatzes im Reaktionsgefäss während des Reaktiveindampfens (Schritt b) verhindern werden kann. Dies trägt zusammen mit der verlängerten Hydrolyse-Kondensationsreaktionszeit von mindestens 16 Stunden dazu bei, dass beim erfindungsgemäßen Verfahren mindestens 70%, vorzugsweise mindestens 80% und ganz besonders bevorzugt mindestens 90% des gesamten Reaktionsansatzes versponnen werden kann.

Schritt (b) wird so lange durchgeführt, bis eine einphasige Lösung mit einer Viskosität im Bereich von 0,5 bis 2 Pa · s bei einer Scherrate von 10 s⁻¹ bei 4 °C, vorzugsweise ca. 1 Pa · s (Messung bei 4 °C, Scherrate 10 s⁻¹), erzeugt wird.

In der Erfindung wird die Verfolgung des Reaktionsforschritts in Schritt b) über die Viskosität erfolgen.

Die aus der Hydrolyse-Kondensations-Reaktion in Schritt b) resultierende homogene und einphasige Lösung wird nachfolgend abgekühlt und quantitativ und optional ohne Filtration einer kinetisch kontrollierten Reifung unterworfen werden.

Die Reifung (Schritt c)) wird erfindungsgemäß bei einer Temperatur von 2 °C bis 4 °C (z.B. in einem Kühlschrank) durchgeführt. Besonders bevorzugt wird die Reifung bei 4 °C durchgeführt. Durch die niedrige Temperatur kann während der Reifezeit kinetisch kontrolliert, eine weitere Kondensation ablaufen. In diesem Gemisch können Oligomere und/oder Polymere Siloxane und/oder Silanole gebildet werden. Die Oligomere und/oder Polymere können auch über Wasserstoffbrückenbindungen aggregieren. Nach der Reifung kann erfindungsgemäß eine strukturviskose homogene einphasige Sol-Masse erhalten werden. Vorteilhafterweise kann erfindungsgemäß daher die konkurrierende Ausbildung eines dreidimensionalen polymeren Gelnetzwerkes weitestgehend unterdrückt werden. Es kann daher eine homogene Sol-Masse gewonnen werden, die keine feste zweite Phase, insbesondere weitestgehend keine Gelphase, aufweist.

Die Reifezeit in Schritt d) kann erfindungsgemäß von 3 Tagen bis 4 Wochen, bevorzugt mindestens 10 Tage, bevorzugter zwischen 14-40 Tagen, beispielsweise zwischen 14 und 28 Tagen, noch bevorzugter mindestens 25 Tage und - speziell bei der Verwendung der erfindungsgemäßen Materialien für die Wundbehandlung - zwischen 25 und 40 Tage betragen.

Erfindungsgemäß bevorzugt weist das in Schritt d) erhaltene Sol eine Viskosität zwischen 30 und 100 Pa·s (Scherrate 10 s⁻¹ bei 4 °C) mit einem Verlustfaktor (bei 4 °C, 10 s⁻¹, 1% Deformation) von 2 bis 5 bevorzugt von 2,5 bis 3,5 (der Verlustfaktor ist der Quotient aus viskosem zu elastischem Anteil der dynamischen Viskosität). Diese Bedingungen für die Reifung sind insbesondere bevorzugt, wenn das Kieselsol nach Schritt d) zu einer Faser versponnen werden soll.

Zur Herstellung des Kieselsol-Materials mit mindestens einem therapeutisch aktiven Wirkstoff wird in einem oder mehreren der Schritte a) bis d), vorzugsweise in einem oder mehreren der Schritte a) bis c) mindestens ein therapeutisch aktiver Wirkstoff dazugegeben.

Erfindungsgemäße, für die Herstellung des Kieselsol-Materials vorzugsweise verwendete therapeutisch aktive Wirkstoffe sind insbesondere ausgewählt aus der Gruppe der Analgetika, Anästhetika, Antiseptika, Hämostyptika, gerinnungshemmende Verbindungen, Antihistaminika, antiphlogistische Verbindungen, pflanzliche wundheilungsfördernde Substanzen oder Substanzgemische, Impfstoffe (z.B. gegen toxische Wundinfektionen), Wachstumsfaktoren, regenerationsunterstützende Proteine wie beispielsweise Collagen, Enzyme, Enzyminhibitoren, insbesondere Protease-Inhibitoren wie beispielsweise alpha-1-Antichymotrypsin und Prolastin, Vitamine oder Provitamine, Carotinoide, hautpflegende Verbindungen, Kontrazeptiva sowie Kombinationen hiervon.

Als erfindungsgemäße Analgetika werden Opioid-Analgetika, basierend auf Prototypen wie Morphin, Fentanyl und Methadon wie beispielsweise Burprenorphin, und Nichtopioid-Analgetika (wie Nikotinerge Analgetika wie beispielsweise Epibatidin; saure antiphlogistische und antipyretische Analgetika (NSAID - Non-steroidal anti-inflammatory drugs = nichtsteroidale Entzündungshemmer) wie Salicylsäurederivate wie beispielsweise Acetylsalicylsäure (ASS), Methylsalicylat oder Diflunisal, Phenylessigsäurederivate wie beispielsweise Diclofenac, 2-Phenylpropion-säurederivate wie beispielsweise Ibuprofen oder Naproxen; nicht-saure Analgetika wie 4-Aminophenol-Derivate wie beispielsweise Paracetamol, Pyrazolone wie beispielsweise Metamizol oder Phenazon, Oxicame wie beispielsweise Meloxicam oder Piroxicam; sonstige Nichtopioid-Analgetika wie beispielsweise Flupirtin) verstanden.

Erfindungsgemäß bevorzugte Anästhetika sind Lokalanästhetika vom Amid- oder Ester-Typ, insbesondere Lidocain, Tetracain, Bupivacain, Prilocain, Mepivacain, Etidocain sowie Procain und Benzocain.

Erfindungsgemäß bevorzugte Antiseptika sind ausgewählt aus der Gruppe der quartären Ammoniumverbindungen wie beispielsweise Benzalkonium Cetrimid, Cetylpyridiniumchlorid und Octenidin; iodhaltige Verbindungen wie beispielsweise lod, lod-Povidon; halogenierte Verbindungen wie beispielsweise Triclosan und Chlorhexidin; Chinolin-Derivate wie beispielsweise Oxichinolin; Phenol Derivate wie beispielsweise Resorcinol, Triclosan, Hexachlorophen; Quecksilberhaltige Verbindungen wie Merbromin und Thiomersal; antimikrobielle Metalle wie beispielsweise Silber, Kupfer oder Zink sowie deren Salze, Oxide oder Komplexe in Kombination oder alleine; Benzoesäure, Benzoylperoxid und/oder Biguanide, insbesondere PHMB.

Als Antiseptikum können erfindungsgemäß auch solche Verbindungen verwendet werden, die eine germizide, bakterizide (z.B. Antibiotika), bakteriostatische (z.B. Antibiotika), bakteriolytische (z.B. Antibiotika), fungizide, viruzide, virustatische, anit-parasitäre und/ oder allgemein mikrobiozide Wirkung aufweisen.

Erfindungsgemäß bevorzugte Hämostyptika sind ausgewählt aus der Gruppe von Thrombin, Fibrin, Fibrinogen, Faktor-VIII-Konzentrat, Vitamin K, PPSB, Protamin, Antifibrinolytika wie beispielsweise Tranexamsäure und Aminokapronsäure.

Erfindungsgemäß bevorzugte gerinnungshemmende Verbindungen sind ausgewählt aus der Gruppe von Heparin, Cumarine, Thrombozytenaggregationshemmer wie beispielsweise Acetylsalicylsäure, Cyclooxygenase (COX)-Hemmer, Clopidogrel, Tirofiban; Fibrinolytika wie beispielsweise Streptokinase, Urokinase, und Alteplase.

Erfindungsgemäße Antihistaminika sind ausgewählt aus der Gruppe der Ethylendiamine wie beispielsweise Mepyramin (Pyrilamin), Tripelennamin (Pyribenzamin), Antazolin, Dimetinden, (Bamipin); der Ethanolamine wie beispielsweise Diphenhydramin, Carbinoxamin, Doxylamin, Clemastin; der Alkylamine wie beispielsweise Pheniramin, Chlorphenamin (Chlorpheniramin), Dexchlorphenamin, Brompheniramin, Triprolidin; der Piperazine wie beispielsweise Hydroxyzin, Meclozin; der trizyklischen Antihistaminika wie beispielsweise Promethazin, Alimemazin (Trimeprazin), Zyproheptadin und Azatadin; Acrivastin, Astemizol, Cetirizin, Ebastin, Fexofenadin, Loratadin, Mizolastin, Terfenadin; Azelastin, Levocabastin, Olopatadin, Epinastin, Levocetirizin, Desloratadin, Fexofenadin Thioperamid und JNJ7777120.

Erfindungsgemäß bevorzugte antiphlogistische Verbindungen sind ausgewählt aus der Gruppe der nichtsteroidalen Antiphlogistika/Antirheumatika wie beispielsweise Acetylsalicylsäure , Diclofenac, Diflunisal, Flurbiprofen, Ibuprofen, Indometacin, Ketoprofen, Mefenaminsäure , Metamizol, Naproxen, Oxyphenbutazon, Phenylbutazon, Phenazon, Piroxicam, Propyphenazon, Salicylamid, Tiaprofensäure, Tenoxicam, Tolfenaminsäure; Glukokortikoide wie beispielsweise Clobetasolpropionat, Triamcinolonacetonid, Betamethasonvalerat, Dexamethason, Prednisolon, Prednison, Hydrocortison, Hydrocortisonacetat, Fluticason, Budesonid; Sonstige Antiphlogistika wie beispielsweise Montelukast oder pflanzliche Antiphlogistika-Extrakte aus Kamille, Ringelblume, Arnika.

Als pflanzliche wundheilungsfördernde Substanzen oder Substanzgemische oder Pflanzenextrakte sind im Zusammenhang mit der vorliegenden Erfindung insbesondere, Hamamelis-Extrakte z.B. Hamamelis virgina, Calendula-Extrakt, Aloe-Extrakt z.B. Aloe vera, Aloe barbadensis, Aloe feroxoder oder Aloe vulgaris, Grüntee- Extrakte, Meeresalgen-Extrakt z.B. Rotalgen- oder Grünalgen-Extrakt, Avocado-Extrakt, Myrre-Extrakt z.B. Commophora molmol, Bambus-Extrakte sowie Kombinationen hiervon zu verwenden. Hierbei sind erfindungsgemäß besonders Extrakte der Blätter, Blüten, Stengel oder Wurzeln der Pflanzen oder Kombinationen hiervon zu verstehen.

Als erfindungsgemäße Wachstumsfaktoren sind insbesondere zu nennen: aFGF (Acidic Fibroplast Growth Factor), EGF (Epidermal) Growth Factor), PDGF (Platelet Derived Growth Factor), rhPDGF-BB (Becaplermin), PDECGF (Platelet Derived Endothelial Cell Growth Factor), bFGF (Basic Fibroplast Growth Factor), TGF α (Transforming Growth Factor alpha), TGF-β (Transforming Growth Factor beta), KGF (Keratinocyte Growth Factor), IGF1/IGF2 (Insulin-Like Growth Factor) VEGF (Vascular Endothelial Growth Factor) und TNF (Tumor Necrosis Factor).

Als erfindungsgemäße Vitamine oder Provitamine sind insbesondere die fettlöslichen oder wasserlöslichen Vitamine Vitamin A, Gruppe der Retinoide, Provitamin A, Gruppe der Carotenoide, insbesondere β-Carotin, Vitamin E, Gruppe der Tocopherole, insbesondere α-Tocopherol, β-Tocopherol, γ-Tocopherol, δ-Tocopherol und α-Tocotrienol, β-Tocotrienol, γ-Tocotrienol und δ-Tocotrienol, Vitamin K, Phyllochinon insbesondere Phytomenadion oder pflanzliches Vitamin K, Vitamin C, L-Ascorbinsäure, Vitamin B 1, Thiamin, Vitamin B2, Riboflavin, Vitamin G, Vitamin B3, Niacin, Nikotinsäure und Nikotinsäureamid, Vitamin B5, Pantothensäure, Provitamin B5, Panthenol oder Dexpanthenol, Vitamin B6, Vitamin B7, Vitamin H, Biotin, Vitamin B9, Folsäure sowie Kombinationen hiervon geeignet.

Erfindungsgemäß bevorzugte hautpflegende Verbindungen sind insbesondere Antioxidantien, Lichtschutzmittel, Insektenrepellentien, ätherische Öle, Moisturizer, Parfüme und/oder Coenzym Q10.

Erfindungsgemäße therapeutische aktive Wirkstoffe die einzeln oder als Gemisch verschiedener therapeutisch aktiver Wirkstoffe eingesetzt werden können, sind insbesondere zu 0,01 bis 40 Gew.-%, bevorzugt zu 0,01 bis 20 Gew.% und ganz besonders bevorzugt zu 0,1 bis 10 Gew.-% bezogen auf das Gewicht des Kieselsol-Materials in der Zusammensetzung enthalten.

Soll die Faser/Vliese für die Wundheilung eingesetzt werden, so weist das in Schritt d) erhaltene Sol vorzugsweise eine Viskosität von 35 bis 75 Pa·s (Scherrate 10 s⁻¹ bei 4 °C) und noch bevorzugter von 35 bis 45 Pa·s (Scherrate 10 s⁻¹ bei 4 °C) vorzugsweise bei einem Verlustfaktor (bei 4 °C, 10 s⁻¹, 1% Deformation) von 2,5 bis 3,5 auf.

Ein zu hoher Verlustfaktor bedeutet eine zu hohe Elastizität des Materials, die z.B. der Ausbildung eines stabilen Fadens bei der Verspinnung entgegensteht (Vergelung, Reißen des Fadens). Bei zu niedrigem Verlustfaktor ist das Material so fließfähig, dass eine stabile Fadenbildung nicht möglich ist (Tropfen).

Die Bedingungen bei der Reifezeit können variieren, sofern das erfindungsgemäße Kieselsol anstatt zu einer verspinnbaren Faser nachfolgend zu einem Pulver verarbeitet werden soll. Vorzugsweise beträgt die dynamische Viskosität am Ende von Schritt (d) in diesem Fall etwa 60 Pa • s (Scherrate 10 s⁻¹ bei 4 °C).

Im Fall der Verarbeitung des Kieselsols zu einem Monolith beträgt die dynamische Viskosität am Ende von (d) vorzugsweise größer gleich 70 Pa • s (Scherrate 10 s⁻¹ bei 4 °C). Wenn das Kieselsol zur Beschichtung von Körpern oder Oberflächen verwendet werden soll, liegt die dynamische Viskosität je nach gewünschter Schichtdicke bei kleiner gleich 10 Pa • s (Scherrate 10 s⁻¹ bei 4 °C).

Vorzugsweise kann die erhaltene Sol-Masse zumindest annähernd quantitativ in weitere Herstellungsschritte und/ oder -prozesse für biologisch degradierbare und/oder resorbierbare Kieselgel-Materialien eingesetzt werden. Bevorzugt ist das in Schritt d) erhaltene Sol spinnbar. In einem weiteren Schritt e) kann erfindungsgemäß ein Spinnprozess vorgesehen sein.

Ein solcher Spinnprozessschritt kann unter üblichen Bedingungen durchgeführt werden, wie zum Beispiel in DE 196 09 551 C1 und DE 10 2004 063 599 A1 beschrieben.

Hierbei wird dass Sol z. B. über einen Druckbehälter durch eine Düsenplatte mit Einzeldüsen ausgeblasen (Druck im Behälter 1-100 bar, vorzugsweise 20 bis 30 bar).

Der Spinnschacht hat üblicherweise eine Länge von 1-5 m vorteilhafterweise 2 m. Das Klima im Spinnschacht wird bzgl. Temperatur und Feuchtigkeit kontrolliert eingestellt. Bevorzugt sind Temperaturen zwischen 20 °C und 30 °C und -5 bis 10 °C Taupunkt beziehungsweise Feuchtigkeit von 20 bis 40% relativer Feuchte, bevorzugt 20-25% relative Feuchte und besonders bevorzugt etwa 20% relative Feuchte.

Die Fasern sind nach dem Durchfallen durch den Spinnschacht formstabil und werden auf einen Changiertisch abgelegt. Die Maschenweite der so entstehenden Fasergelege wird u. a. über die Changiergeschwindigkeiten eingestellt. Diese liegt bei einigen cm/s. Durch eine zwei-Achsenbewegung entsteht so ein engmaschiges Fasergelege (Vlies), bei dem, bezogen auf TEOS als Si enthaltende Ausgangsverbindung, i.d.R. noch über 25 bis 33 %. der Ethoxy-Gruppen vorhanden sind.

Speziell bei der Verwendung der erfindungsgemäßen Materialien für die Wundbehandlung ist das Flächengewicht des Fasermaterials vorzugsweise mindestens 90 g/m², und besonders bevorzugt mindestens 150 g/m². Die Dicke der Wundauflage (bestehend aus dem gesponnenen Vlies) ist bevorzugt mindestens 0,8 mm und besonders bevorzugt mindestens 1,5 mm. Der Faserdurchmesser ist vorzugsweise mindestens etwa 45 µm.

Die aus dem erfindungsgemäßen Verfahren resultierenden Kieselgel-Fasermaterialien und Produkte, also beispielsweise Fäden, Fasern, Vliese und/oder Gewebe, weisen eine hervorragende biologische Degradierbarkeit und biologisches Resorptionsvermögen auf.

Ein weiterer erfindungsgemäßer Vorteil ist, dass erfindungsgemäß erzeugte Kieselgel-Fasermaterialien, gegenüber Fasern, die nach dem Verfahren der DE 196 09 551 C1 erhalten wurden, deutlich verbesserte Werte in Cytotoxizitätstests in Tests in Gegenwart von L929-Mausfibroplasten aufweisen (siehe Beispiel 1 und Vergleichsbeispiel). Produkte, die aus dem erfindungsgemäßen Kieselsol-Material erzeugt wurden, zeichnen sich daher durch eine besonders gute biologische Verträglichkeit aus. Die erfindungsgemäßen Fäden, Fasern oder Vliese können insoweit vorteilhaft als biodegradierbare und/oder biologisch resorbierbare Materialien und Produkte in der Humanmedizin oder Medizintechnik eingesetzt werden.

Die Fasern und Vliesen mit mindestens einem therapeutisch aktiven Wirkstoff können die verbesserten Wundheilungseigenschaften der Fasern und Vliesen alleine unterstützen bzw. noch verbessern. Insbesondere können die erfindungsgemäßen Materialien daher vorteilhaft im Bereich der Wundbehandlung und Wundheilung verwendet werden. Fäden können beispielsweise als chirurgisches Nahtmaterial oder als Verstärkungsfasern eingesetzt werden. Erfindungsgemäße Faservliese können besonders vorteilhaft bei der Versorgung von oberflächlichen Wunden verwendet werden.

Die erfindungsgemäß hergestellten Fasern oder Vliese können insoweit vorteilhaft als biologisch resorbierbare und/oder bioaktive Materialien in der Humanmedizin und/oder der Medizintechnik eingesetzt werden. Insbesondere können die erfindungsgemäß hergestellten Materialien vorteilhaft im Bereich der Wundbehandlung und Wundheilung verwendet werden. Fasern können beispielsweise als chirurgisches Nahtmaterial oder als Verstärkungsfasern eingesetzt werden. Vliese können besonders vorteilhaft bei der Versorgung von oberflächlichen Wunden, bei der Filtration von Körperflüssigkeiten (z.B. Blut) oder im Bereich der Bioreaktoren als Anzuchthilfe verwendet werden.

Eine weitere Ausführungsform kann ein Drug Delivery System und/oder eine Arzneimittelformulierung, ein Mikropulver und/oder ein Nanopulver sein. Es können selbstverständlich weitere, der jeweiligen Anwendung angepasste Substanzen und/oder Hilfsstoffe in der endgültigen Formulierung (Pulver) vorhanden sein. Die Partikel eines erfindungsgemäßen Mikropulvers haben vorzugsweise eine Größe (einen mittleren Durchmesser) von 0,01 µm bis 100 µm, insbesondere 0,1 µm bis 20 µm. Die Nanopulver-Partikel haben in der Regel eine Größe (einen mittleren Durchmesser) von ≤ 100 nm.

In einer weiteren Ausgestaltung wird das Kieselsol in eine Form gegossen. Nach der Trocknung kann auf diese Weise ein Monolith erhalten werden. Solche Monolithe können in Form von massiven Implantaten als Wirkstoff Zulieferungs-System (Drug Delivery System) beispielsweise subkutan eingesetzt werden. Sie können beispielsweise als Depot für Kontrazeptiva eingesetzt werden und über einen längeren Zeitraum den Wirkstoff freigeben. Solche erfindungsgemäßen Implantate weisen eine gute biologische Verträglichkeit auf. Die Monolithe können vorzugsweise einen Durchmesser von ≥ 0,5 mm aufweisen. Alternativ können die Monolithe auch zu Pulver zerkleinert und gemahlen werden.

Nach einer weiteren Ausgestaltung können hochviskose Sole, insbesondere Hydrogele, durch das erfindungsgemäße Kieselgel ergänzt oder ersetzt werden. Generell werden Hydrogele vielfach in der Versorgung von großflächigen Wunden (Wundbehandlung und Wundheilung) eingesetzt. Vorteilhafterweise kann durch den Zusatz des Kieselsols die biologische Verträglichkeit und damit die Wundheilung verbessert werden. Die Hydrogele können insoweit vorteilhaft als biologisch resorbierbare und/oder bioaktive Produkte in der Medizin, insbesondere Humanmedizin oder Medizintechnik eingesetzt werden.

Offenbart ist weiter ein Verfahren zur *in-vitro*-Vermehrung von Zellen, wobei eine Fasermatrix aus einer Faser als Zellstützsubstanz und/oder Leitstruktur für die von den Zellen gebildete extrazelluläre Matrix dient bzw. den Zellen die Möglichkeit gibt, eine räumliche Anordnung zu finden, die es den Zellen erlaubt, sich zu vermehren und/oder ihre genetisch determinierte Differenzierung zu erreichen. Die Vorteile des

Verfahrens ergeben sich exemplarisch aus Beispiel 3.

Als Zellen können beispielsweise undifferenzierte pluripotente Stammzellen oder gentechnisch veränderter oder nativer differenzierter Zellen verschiedener Differenzierungsarten und -grade verwendet werden.

Die auf die Fasermatrix aufzubringenden Zellen haften an die Matrix an oder vermehren sich vornehmlich zweidimensional auf dieser Matrix um zusammen eine extrazelluläre Matrix bzw. Botenstoffe (Hormone) zu bilden. Die Fasermatrix bildet vorzugsweise ein Flächenelement, insbesondere in Form eines Vlieses oder Gewebes aus erfindungsgemäßen Fasern. Vorzugsweise ist diese Fasermatrix porös, so dass die ein-/aufgebrachten Zellen sie penetrieren, eine dreidimensionale Verteilung annehmen und entsprechend ihrer genetisch determinierten oder durch hinzu gegebene Differenzierungsfaktoren induzierten Differenzierung ein räumliches Gewebe- und Organwachstum auslösen können bzw. Botenstoffe freisetzen. In einer alternativen Ausführungsform ist die Matrix als dichtes, von den ein-/aufgebrachten Zellen nicht penetrierbares Fasernetz mit der Möglichkeit der zweidimensionalen Zellverteilung und der gleichzeitigen Möglichkeit eines dreidimensionalen Gewebe- und Organwachstums im Sinne eines "Composite grafts" ausgebildet.

Das *in-vitro*-Vermehrungsvefahren dient vorzugsweise der *in-vitro*-Herstellung von Zellverbunden, Geweben und/oder Organen.

Ein weiterer Gegenstand betrifft einen Zellenverbund, Gewebe und/oder Organe, herstellbar nach dem oben beschriebenen Verfahren. Ein solcher/s Zellverbund, Gewebe und/oder Organe eignet sich beispielsweise als *in vitro*-Modell für Medikament-Gewebe-Organinteraktionen. Zur Herstellung von Geweben außerhalb des menschlichen Körpers kommen vielfältige Verfahren zur Anwendung, die unter dem weitläufigen Begriff "tissue engineering" zusammengefasst werden. Hierzu werden je nach Gewebeart Zellen aus ihrem bestehenden Gewebeverbund isoliert und zur Vermehrung gebracht. Hiernach werden die Zellen entweder auf flächige Materialien unterschiedlicher Konsistenz aufgebracht oder in poröse bzw. gelartige Materialien eingebracht, hierdurch die Gewebereifung induziert und gegebenenfalls durch Differenzierungsfaktoren stimuliert. Die Gewebereifung kann außerhalb oder innerhalb des Körpers erfolgen. Die erfindungsgemäße Fasermatrix hat dabei den Vorteil, dass sie biologisch degradierbar und/oder biologisch resorbierbar ist, jedoch - wie Beispiel 3 zeigt - trotzdem bei der in-vitro-Vermehrung über einen gewissen Zeitraum ihre 2- bzw. 3-dimensionale Form nahezu beibehält. Ein weiterer Gegenstand betrifft demnach einen Zellenverbund, Gewebe und/oder Organe mit einer Fasermatrix aus Polykieselsäure, bevorzugt hergestellt aus den Fasern, wobei die biologisch degradierbare und/oder biologisch resorbierbare Fasermatrix nach einer Zeitperiode von 4 Wochen nach erstmaliger in-vitro Zellbesiedelung zu mindestens 60%, vorzugsweise zu mindestens 70% und besonders bevorzugt zu mindestens 80% identisch mit der ursprünglichen 2- bzw. 3- dimensionale Form der Fasermatrix ist. Beispielsweise degradiert und/oder resorbiert die Fasermatrix bei einer solchen Ausführungsform vorzugsweise erst nach Auf-/Einbringen des Zellverbundes, Gewebes und/oder Organs auf/in einen tierischen oder menschlichen Körper.

Je nach Zellart müssen die Zellen entweder zuvor durch enzymatischen Verdau oder durch mechanische Trennung aus ihrem Matrixverbund gelöst werden oder durch Auf- oder Einbringen auf/in ein Nährmedium unter physiologischen Bedingungen zum Wachstum angeregt werden. Die oben genannte Fasermatrix fungiert hierbei als Leitstruktur für das Zellwachstum oder als Leitstruktur für die Akkumulation extrazellulärer Matrix- und Gewebebestandteile. das Faser-Material kann in verschiedenen Anordnungen zur Anwendung kommen. Welche Anordnung zu wählen ist, weiß der Fachmann an Hand des herzustellenden (Zell-)Gewebes zu bestimmen. Die in Betracht kommenden Anordnungen sind die folgenden:
1) als Flächenelement, d.h. als dichtes Fasernetz, das zwar eine über die Dimension der aufgebrachten Zellen hinausgehende, aber doch nur eine begrenzte Penetration ermöglicht (d.h. die durchschnittliche Größe der Löcher/Faser-bzw. Netzzwischenräume ist keinesfalls größer, bevorzugt sogar kleiner als die durchschnittliche Größe der zu kultivierenden Zellen; somit können die Zellen zwar "in die Fasern einwachsen", dies aber nur derart, dass sie auf der Unterlage der Fasern gut haften), mit der im wesentlichen alleinigen, zumindest aber vornehmlichen Möglichkeit der zweidimensionalen Zellverteilung und einem flächigen Zell, -Gewebe und Organwachstum.
2) als dreidimensionales Raumelement, d.h. als poröses, von den Zellen penetrierbares Fasernetz (d.h. die durchschnittliche Größe der Löcher/Faser bzw. Netzzwischenräume ist keinesfalls kleiner, bevorzugt sogar größer als die durchschnittliche Größe der zu kultivierenden Zellen) mit der Möglichkeit der dreidimensionalen Zellverteilung und einem räumlichen Zell, -Gewebe-und Organwachstum; 3) als Kombination von 1) und 2) im Sinne eines "composite graft" oder Organes durch Kombination von Zellen, Geweben bzw. Organen und Oberflächen-Hüllgewebe (z.B. Organkapsel);
3) Diese Variante kommt in Betracht für Gewebestrukturen, die aus mehreren Zellarten zusammengesetzt sind. Beispielsweise bestehen Gefäße aus Endothel und Bindegewebe, wobei das Endothel mit flächiger Struktur zur Auskleidung eines Blutgefäßes dient, während das Bindegewebe als Stützsubstanz des Gefäßes fungiert und die dreidimensionale Hohlstruktur bildet. Durch die Kombination von 1) als Flächenelement für das Wachstum von Endothel und 2) als dreidimensionales Raumelement für das Wachstum von Bindegewebe kann letztendlich ein Gefäß rekonstruiert werden.

Nachfolgend werden einige Gewebe- bzw. Zelltypen aufgelistet, die für die Vermehrung/Herstellung mittels einer der drei Varianten besonders geeignet sind und demnach bevorzugt sind.

Für die Anwendung 1) vorzugsweise die folgenden Gewebe: Epithel, Endothel, Urothel, Mukosa, Dura, Bindegewebe; und vorzugsweise die folgenden Zellen: pluripotente Stammzellen, Chondrozyten (Knorpel; zur Chondrozyten-Vermehrung braucht man ein zweidimensionales Medium, zur Chondrozyten-Differenzierung und Knorpelmatrix-Bildung hingegen braucht man ein dreidimensionales Medium. Hier sind bezüglich Knorpel nur die Zellen gemeint, wenn sie dedifferenzieren und sich vermehren. Die Differenzierung folgt in Anwendung 2), Osteozyten (Knochen; entweder zwei oder dreidimensional, hier gilt dasselbe wie für die Chondrozyten), Nervenzellen (Nerven), Haarzellen (Innenohr-Hörorgan) oder deren Vorläuferzellen jeglicher Differenzierungsstufe (z.B. pluripotente Stammzellen).

Für die Anwendung 2) die folgenden Zellen: die für Anwendung 1) beschriebenen Zellen nach ihrer flächigen Vermehrung, organspezifische Zellen (z.B. Hepatozyten, Nephrozyten, Kardiomyozyten, Pankreozyten), Zellen des ZNS mit/ohne endokrine/r Funktion, z.B. Netzhaut, Neurozyten, Zirbeldrüse, dopaminerge Zellen, Gefäß bildende Zellen (z.B. Angiozyten), Zellen mit endo -oder exokriner Funktion (z. B. Inselzellen, Nebennierenzellen, Speicheldrüsenzellen, Epithelkörperchen, Thyrozyten), Zellen des Immunsystems (z.B. Makrophagen, B-Zellen, T-Zellen oder deren Vorläuferzellen jeglicher Differenzierungsstufe wie pluripotente Stammzellen). Die Zellen des Immunsystems werden dreidimensional gezüchtet, weil sie im Gewebe, nach Penetration der Blut-Gewebeschranke auf ein dreidimensionales Gerüst je nach Gewebeart) treffen und dort im Dreidimensionalen ihre Wirkung entfalten.

Für die Anwendung 3) die folgenden Zeilen/Gewebe/Organe: Trachea, Bronchien, Gefäße, Lymphgewebe, Harnröhre, Harnleiter, Niere, Blase, Nebenniere, Leber, Milz, Herz, Gefäße, Schilddrüse, Tonsillen, Speicheldrüsen, Gehirn, Muskel (glatt, quergestreift), Bandscheiben, Meniskus, Herz, Lunge, Galle, Speiseröhre, Darm, Auge.

Eine weitere Anwendungsmöglichkeit des Materials ist die Besiedlung des Materials mit Zellen, die eine endo bzw. exokrine Funktion besitzen und Wirkstoffe (z.B. Hormone, Interleukine, Entzündungsmediatoren, Enzyme) freisetzen, die eine Wirkung im Organismus oder außerhalb des entfalten. Das heißt, das verwendete Material kann, bei seiner Besiedelung mit Zellen mit endo-oder exokriner Funktion auch außerhalb des Körpers zur Herstellung der o.g. Wirkstoffe dienen, die dann über bekannte Verfahren als Arzneimittel dem Körper zur Verfügung gestellt werden. Eine außerhalb des Körpers entfaltete Wirkung kann dazu dienen, Gewebe oder Zellen mit der freigesetzten Substanz zu beeinflussen.

Eine weitere Verwendung der Matrix ist die als bioresorbierbares Bio-Implantat als Leitschiene für die körpereigene Wundheilung unter oder auf Niveau der Haut, Schleimhaut bzw. im Körperinneren im Rahmen von Operationen an Organen und Geweben. Hierzu wird das Material falls möglich durch einen Arzt als Flächenelement oder dreidimensionales Raumelement direkt oder zusammen mit weiteren Substanzen in die Wunde oder Organe/Gewebe, beispielsweise während einer Operation, eingebracht. Die Eigenschaften des verwendeten bioresorbierbaren, anorganischen Materials in Form von Fasern bedingen für die zu züchtenden Zellen eine nur geringe Änderung des Gewebemilieus, insbesondere entsteht kein saures Milieu, mit der Folge, dass eine negative Beeinflussung der Gewebe- und Organdifferenzierung verhindert wird. Weiterhin kommt es, unabhängig vom pH-Wert des Gewebes, zu einem vollständigen Abbau des Materials. Durch den gleichzeitig stattfindenden Gewebe- bzw. Organaufbau findet sich immerzu vitales Gewebe mit der Möglichkeit der Penetration durch antiinfektiöse Medikamente bei ungewollter Besiedlung mit Erregern (Infektion). Darüber hinaus kann die Fasermatrix zusätzlich mit weiteren Wirkstoffen unterschiedlicher Substanzgruppen versetzt werden mit der Möglichkeit einer positiven Beeinflussung der Gewebe- und Organdifferenzierung durch Entfaltung einer aktiven und passiven Wirkung am Ort der Anwendung, aber auch durch Wirkungsentfaltung an einem entfernt liegenden Wirkort. Hierzu zählen die oben genannten therapeutisch aktiven Wirkstoffe insbesondere einerseits antiinfektiöse Wirkstoffe, andererseits aber auch die Wundheilung, die Entzündungsreaktion sowie die Gewebedifferenzierung unterstützende und modulierende Wirkstoffe wie beispielsweise einerseits Wachstumsfaktoren (IGF, TGF, FGF etc.), andererseits Glukocortikoide und Interleukine, aber auch Chemotherapeutika und Immunsuppressiva.

Die verwendeten bioresorbierbaren, anorganischen Fasern ermöglichen eine Anhaftung der zur Anwendung kommenden Zellen mit der Möglichkeit zur Vermehrung der Zellen entlang der Fasern, aber auch mit der Möglichkeit der Ausbildung einer Gewebe- bzw. Organmatrix. Gleichzeitig mit der Vermehrung der Zellen bzw. der Ausbildung einer Gewebe bzw. Organmatrix kommt es zum Abbau der Faserstruktur. Idealerweise wird der Gewebe-, Organ- bzw. Zellaufbau mit der Abbaurate des Fasermaterials durch Variation der Kondensation der Fasern korreliert. Dabei gilt, je geringer der Kondensationsprozess (d.h., die Abspaltung von Wasser und damit die Polykondensation) fortgeschritten ist, um so besser kann das Material abgebaut werden. Der höchste OH-Gehalt und damit die am schnellsten abbaubare Faser erhält man bei frisch gesponnenen Fasern, die anschließend in Ethanol eingelegt sind. Der Kondensationsprozeß wird außerdem von den Spinnparametern beeinflusst, d.h. Abziehrate, Atmosphäre, Spinntemperatur etc. So hergestellte Fasern sind biologisch degradierbar und biologisch resorbierbar und lösen sich in einem einstellbaren Zeitraum von vorzugsweise 2 Wochen bis 10 Wochen auf, wobei die Abbaurate mit der Zahl der Silanol-Gruppen der Faser korreliert ist. Ein weiterer Aspekt betrifft die Verwendung der Zellen, Organe und Gewebe, nachdem sie mit Medikamenten und/oder Wirkstoffen versetzt worden sind, als in *vitro-*Modell für Medikament-Gewebe-Organinteraktionen. Hierdurch können tierexperimentelle Untersuchungen minimiert bzw. vermieden werden.

Ein weiterer Gegenstand betrifft ein Verfahren zur Herstellung eines Hautimplantats, wobei man Hautzellen auf die Oberfläche einer Nährlösung aufbringt und wachsen lässt und auf die Nährlösung ein Flächenelement aus einer Faser aufgelegt.

Weiter betrifft die vorliegende Offenbarung ein Hautimplantat bestehend aus Hautzellen und ein Flächenelement mit Fasern. Ein Flächenelement (vorzugsweise planar) ermöglicht ein flächiges und damit schnelles Wachstum von Hautzellen, gegebenenfalls unter zusätzlichem Einsatz von weiteren infiltrierten Medikamenten, die zu der Faser dazugegeben werden.

Die Erfindung soll mit folgenden Beispielen näher erläutert werden, ohne hierauf beschränkt zu sein.

Alle angegebenen Viskositäten wurden mit Viskosimetern der Firma Anton Paar (Typ Physica MCR300 und MCR301) bei einer Scherrate von 10 s⁻¹ bei 4 °C gemessen.

### Beispiele

### Beispiel 1

### Kieselsol und biologisch resorbierbares und biologisch degradierbares Kieselgelmaterial

Als Edukte für die Hydrolyse-Kondensation wurden 4 Mol TEOS (Tetraethoxysilan) in Ethanol in einem Reaktionsgefäß vorgelegt und 7 Mol Wasser in Form einer 0,01n HNO₃-Lösung zugegeben und unter Rühren miteinander vermischt. Die Mischung wurde über 8 Tage bei Raumtemperatur gerührt. Die Lösung aus der Hydrolyse-Kondensationsreaktion wurde nachfolgend beim Eindampfen und Kondensieren im Becherglas bei 70 °C in eine nahezu Wasser- und Ethanol-freie Lösung überführt. Diese Lösung war einphasig, enthielt keine Feststoffe und wies eine Viskosität von 1 Pa·s (Scherrate von 10 s⁻¹ bei 4 °C) auf. Die Lösung wurde auf 4 °C abgekühlt und bei dieser Temperatur einer Reifung unterworfen. Nach einer Reifezeit von 18 Tagen wurde eine homogene einphasige Sol-Masse mit einer Viskosität von 43 Pa·s (Scherrate 10 s⁻¹ bei 4 °C) erhalten. Die Sol-Masse lag ohne erkennbaren festen Phasenanteil vor. Die homogene Sol-Masse konnte zu Fasern versponnen werden. Sie wird auch als Spinnmasse bezeichnet.

Die Herstellung der Fasern erfolgte in einer üblichen Spinnanlage. Dazu wurde die Spinnmasse in einen auf -15 °C gekühlten Druckzylinder gefüllt, der mit einem Luftdruck von 20 bar beaufschlagt wurde. Die daraus resultierende Kraft presste das Sol durch Düsen, wodurch Fäden geformt wurden. Die Fäden wiesen je nach Düsendurchmesser einen Durchmesser von 5 und 100 µm auf.

Die fließfähigen, honigartigen Fäden fielen durch ihr Eigengewicht in einen unter dem Druckzylinder befindlichen Spinnschacht und reagierten dort zu einer weitgehend festen Form und formstabile Fäden wurden gebildet. Die Fäden waren an ihrer Oberfläche noch reaktiv, so dass sie beim Auftreffen auf einen gegebenenfalls vorgesehenen Chargiertisch an den Berührungsflächen miteinander verkleben konnten. Durch einstellbare Hubzyklen des Changiertisches entstanden weitere Quervernetzungen zwischen den Fasern und ein Vlies wurde gebildet.

Vorteilhafterweise waren die erfindungsgemäß erhaltenen Fäden trockener als unter vergleichbaren Spinnbedingungen gewonnene Fasern, die nach dem Verfahren der DE 196 09 551 C1 gefertigt wurden. Hierdurch wurden bei der nachfolgenden Fertigung von Vliesen erfindungsgemäß geringer vernetzte und daher flexiblere Vliese erhalten.

Das erfindungsgemäß hergestellte Vlies wurde einem cytotoxikologischen Test nach ISO 10993-5 (1999); EN 30993-5 (1994) unterzogen. Nach Extraktion des Vliesmaterials mit DMEM (Dulbecco's modifiziertes Eagle-Medium) wurde das Extrakt steril gefiltert und mit FCS (Fetal Calf Serum; 10% FCS im Extrakt) versetzt. Dieses mit FCS versetzte Extrakt wurde unter sterilen Bedingungen auf L929 Maus Fibroplast Zellen aufgetragen und für 48 h bei 37 °C und einem CO₂ Partialdruck von 5% aufbewahrt.

Triton X 100 wurde als toxische Kontrollsubstanz, das Zell-Kultur-Medium als nicht-toxische Kontrollsubstanz verwendet. Die Zellen wurden zur Bestimmung der Zellzahl fixiert und mit Methylenblau gefärbt. Nach saurer Extraktion des Methylenblaus wurde der Farbstoffgehalt mittels Photometrie erfasst und die Extinktion mit einer Standardkurve verglichen, um die Zellzahl anhand der Farbstoffextinktion zu bestimmen. Die Messung der Zellzahl im Vergleich zur Kontrolle ergab, dass das erfindungsgemäße Kieselgelmaterial keine cytotoxischen Eigenschaften aufwies. Messungen des Proteingehalts (nach alkalischer Lyse und Proteingehaltbestimmung mit der Bradfordmethode) und der Freisetzung von Lactatdehydrogenase (LDH; photometrische Methode) bestätigten die Ergebnisse.

### Vergleichsbeispiel

Unter den gleichen Bedingungen wurden Toxizitätsmessungen mit einem Vliesmaterial durchgeführt, das analog dem Beispiel in der DE 196 09 551 C1 mit einer Hydrolyse-Kondensationszeit von 1,5 h erzeugt wurde. Es konnten hierbei nur 50% des gesamten Reaktionsansatzes versponnen werden. Bei dem hieraus erzeugten Fasermaterial wurde im Cytotoxizitätstest eine Cytotoxizität festgestellt.

### Beispiel 2

In einer weiteren Studie wurden fünf verschiedene Faservliesen (KG211, KG226, AEH06KGF553, AEH06KGF563 und AEHKGF565 mit einem resorbierbaren Kontroll-Wundtherapeutikum (Promogran®) in einer über 3 Monate laufenden Wundheilungsstudie am Meerschweinchen verglichen.

Unterschiede der Faservliesen ergeben sich durch die in der nachfolgenden Tabelle 1 aufgeführten unterschiedlichen Herstellungsparametern.

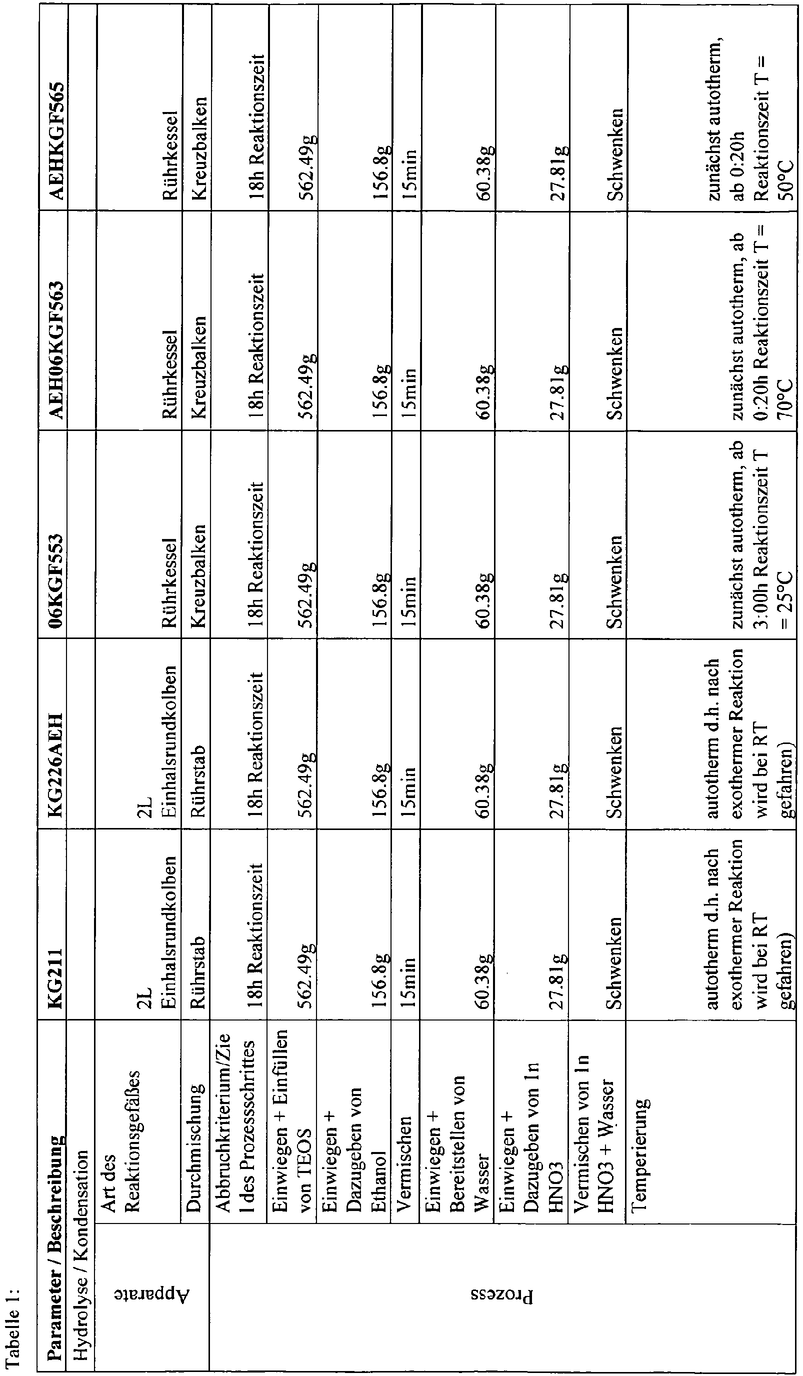

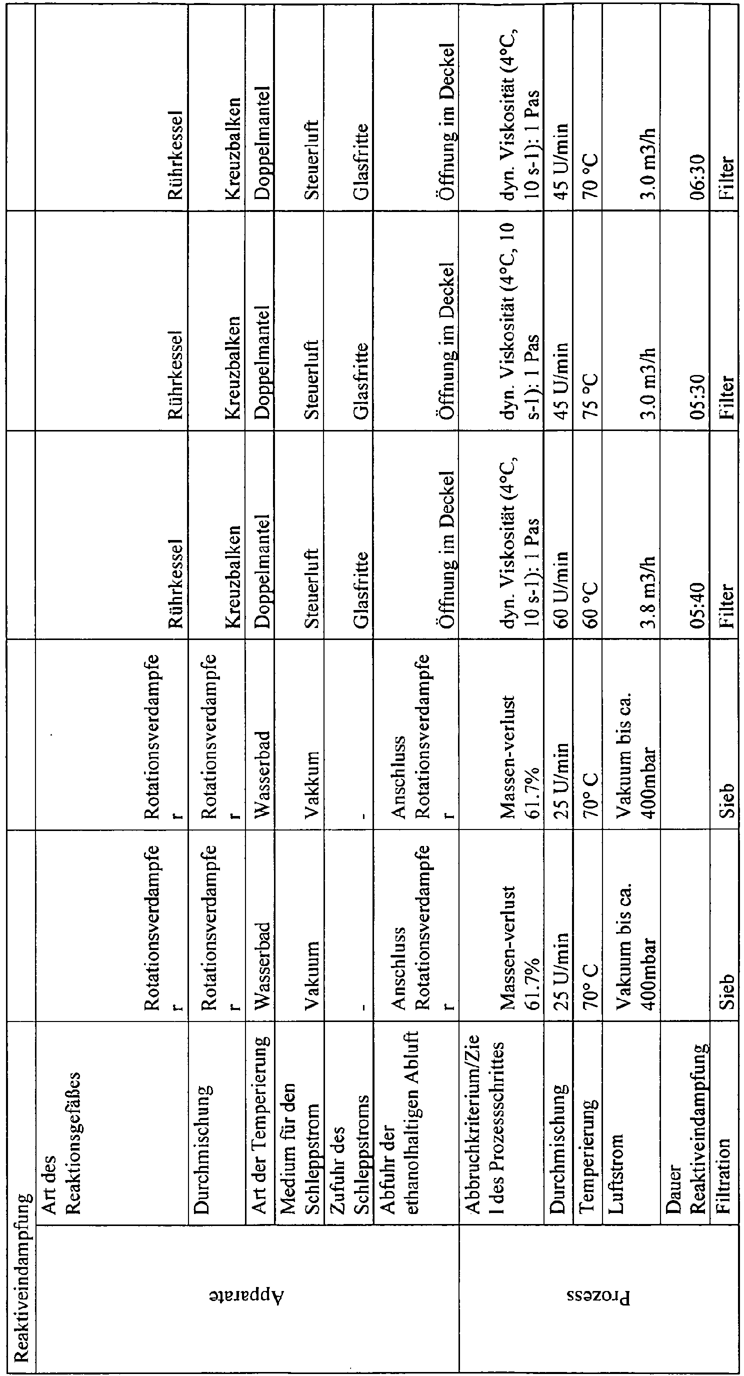

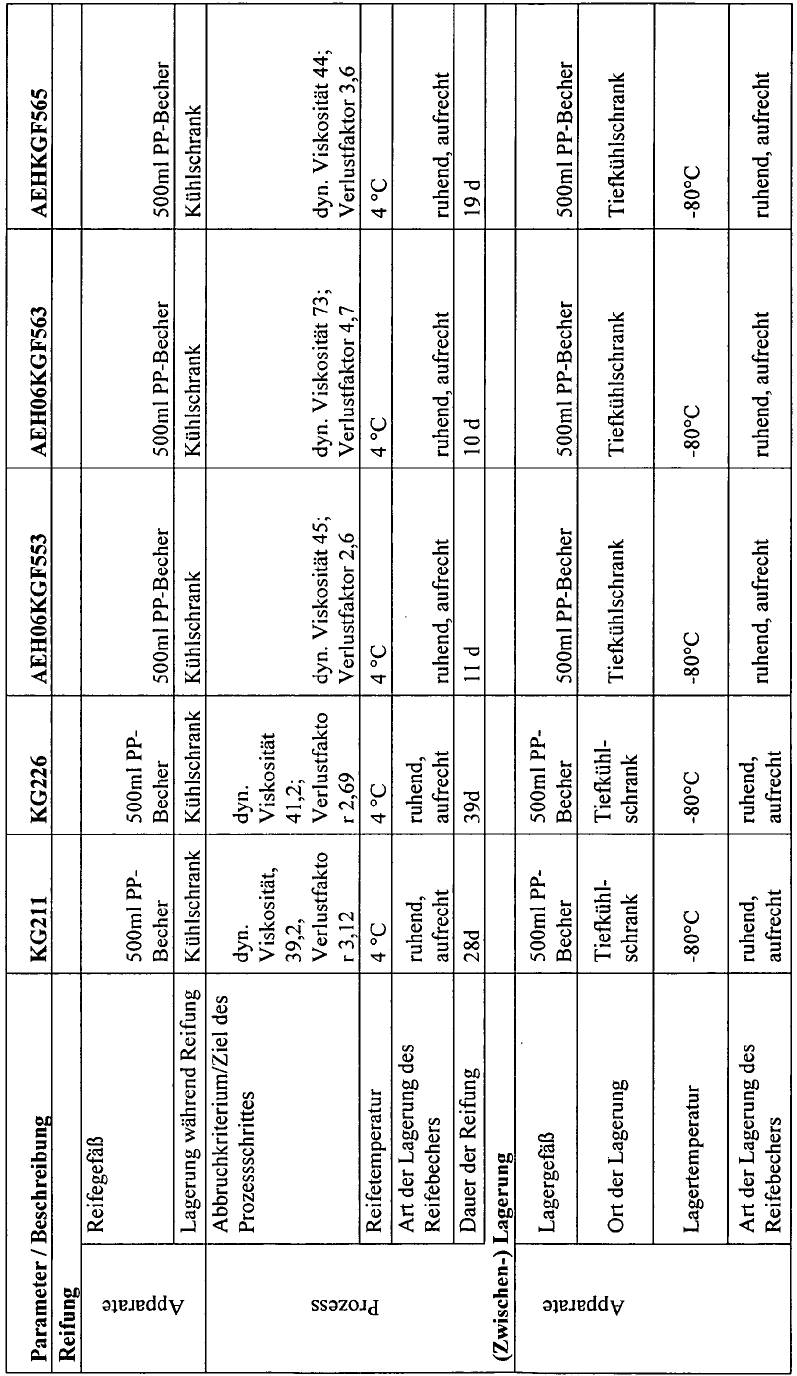

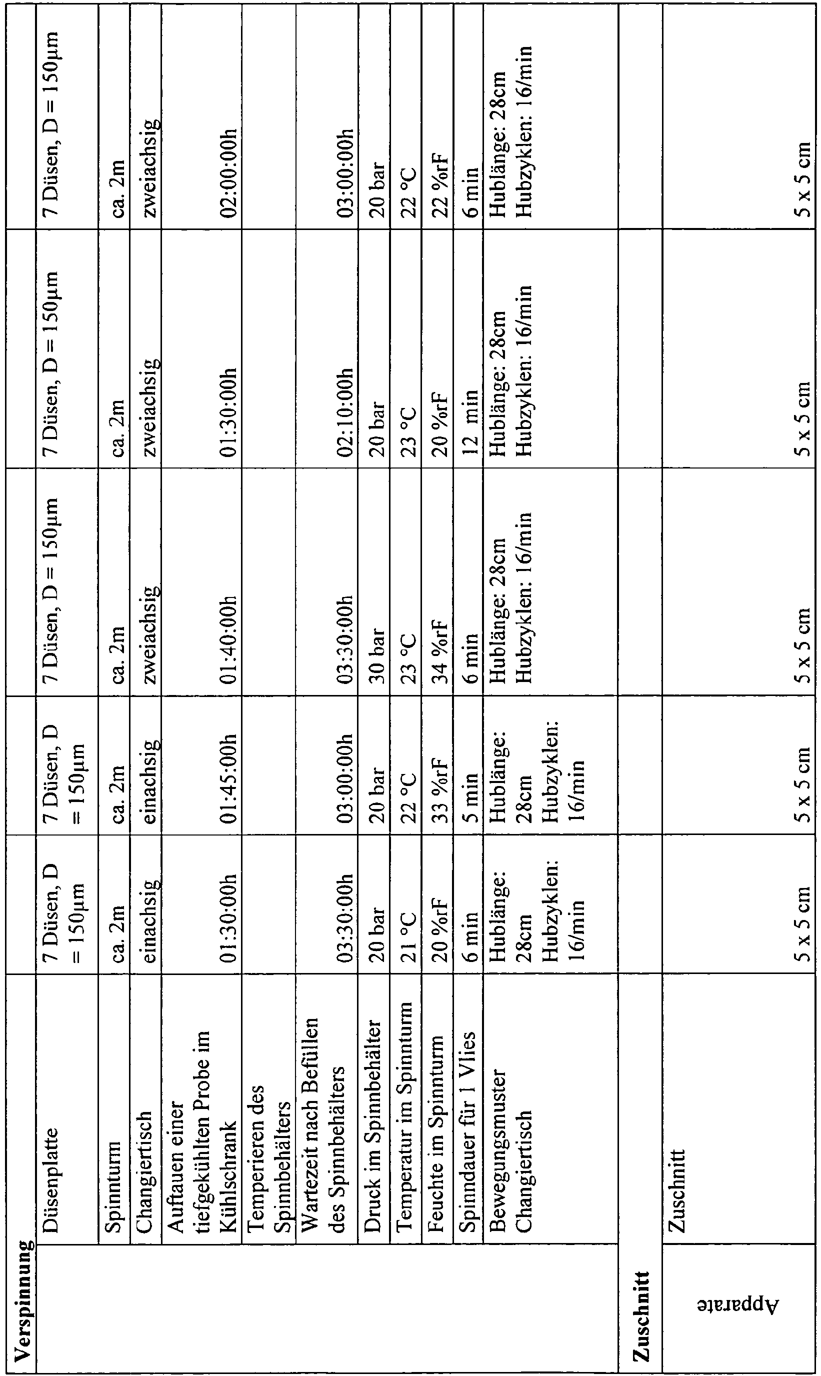

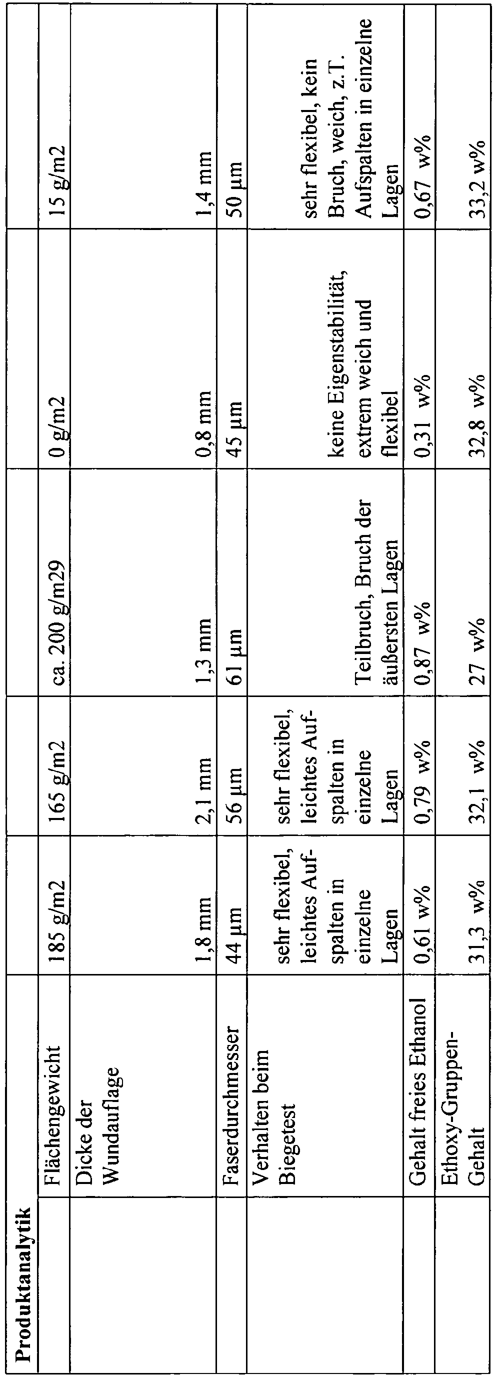

Für die Studie wurden bei 36 Meerschweinchen dermo-epidermale Wunden chirurgisch erstellt. Bei jedem Tier wurden Dermis und Epidermis auf beiden Seiten der Wirbelsäule in einer ungefähren Fläche von 6,25 cm² (2,5 x 2,5 cm) entfernt. Die Wunden wurden durch ein Skalpel erzeugt. Der Panniculus carnosus wurde nicht verletzt. Die Wundauflagen und Promogran® wurden in die jeweiligen Wunden gelegt. Die Materialien wurden mit einem nicht-adhesiven Wundverband (URGOTUL®) und einem semipermeablen adhesiven Poyurethanfilm (TEGADERM® oder OPSITE®) abgedeckt. Eine kohäsive Binde (Gaze und ELASTOPLAST®) schützte die Wundverbände über der Wunde. Jedes Faservlies bzw. das Kontrollmaterial wurde an 5 Tieren, entsprechend 10 Wunden (n = 10) getestet. In verschiedenen Zeitabständen wurde die Wundheilung durch makroskopische, morphometrische und histologische Untersuchungen evaluiert.

Bei allen getesteten Wundauflagen wurde keine lokale Intoleranz beobachtet. Morphometrische Untersuchungen ergaben, dass diejenigen Wunden, die mit Promogran® behandelt wurden einen 50%igen Wundverschluss etwas früher erreichten als die mit den Vliesen behandelten. Um einen vollständigen (100%) bzw. nahezu vollständigen (75%, 95%) Wundverschluss zu erreichen war die Zeit für Promogran® im Vergleich zu den meisten Vliesen jedoch etwas verzögert. 100% Heilung wurde bei KG211 und KG226 im Mittel nach ca. 23 Tagen erreicht, für AEH06KGF553, AEH06KGF563 und AEH06KGF565 im Mittel nach ca. 24 Tagen und für Promogran® erst nach im Mittel 26 Tagen.

Histologische Untersuchungen von KG211 Tieren 28 Tage nach der Generierung der Wunde zeigten eine sehr gute Wundheilung (siehe Figur la). Einzig die lokale Gewebereaktion war noch nicht vollständig stabilisiert, da vereinzelt noch Makrophagen zu beobachten waren. Unabhängig davon war das Granulationsgewebe unauffällig, zeigte eine normale Dicke und war von einer neugebildeten geschlossenen Epithelschicht überdeckt.

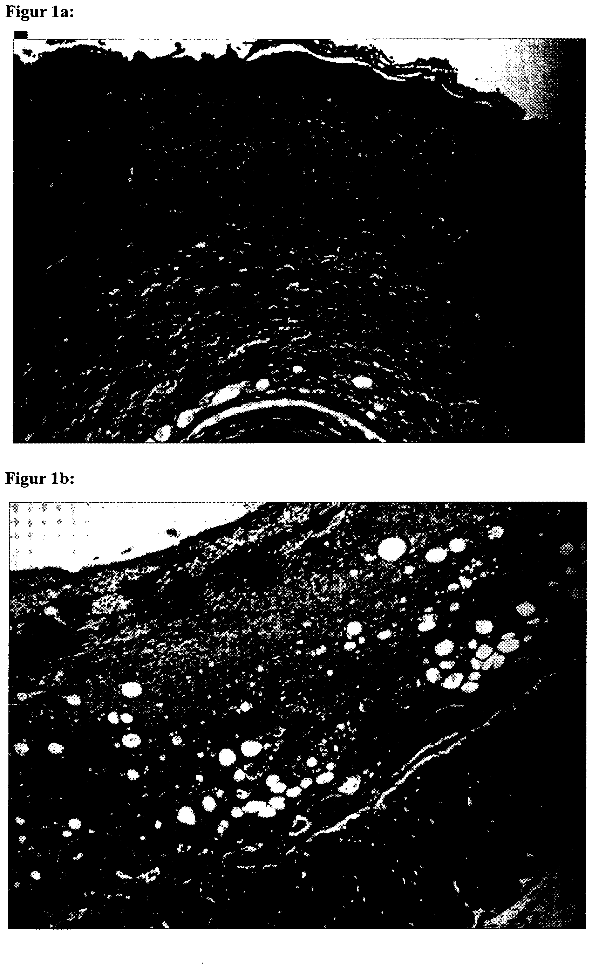

Histologische Untersuchungen der Promogran® Tiere 28 Tage nach Generierung der Wunde zeigten ein stark vakuolisiertes und von polymorphonuklearen Zellen durchzogenes Granulationsgewebe (siehe Figur 1b). Im Gegensatz zu KG211 war das Granulationsgewebe nicht von einer epithelialen Schicht bedeckt.

Die Wundauflagen zeigen demnach verkürzte Wundheilung bei gleichzeitiger Generierung einer besseren Granulationschicht und einer Minimierung inflammatorischer Prozesse im Vergleich zu Promogran® in den ersten 4 Wochen der Wundheilung.

### Beispiel 3

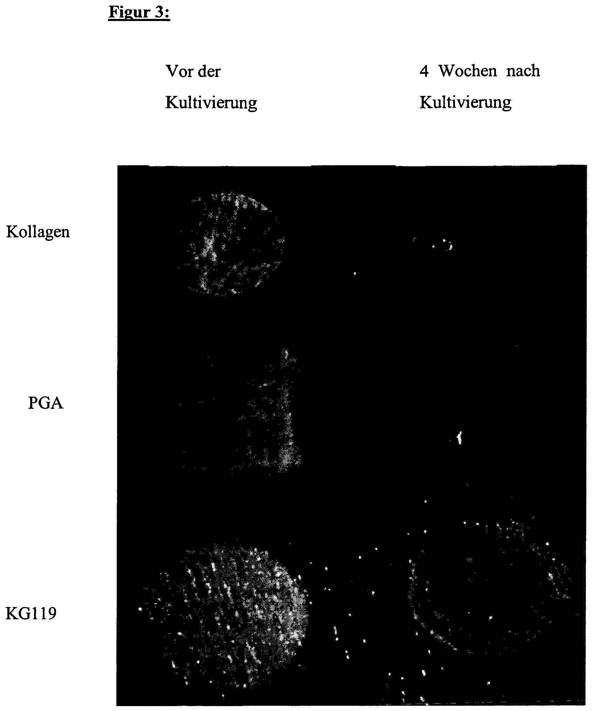

Die Fasermatrix KG119 aus biologisch degradierbaren und/oder biologisch resorbierbaren Fasern als Zellstützsubstanz sowie Kollagen und Polyglykolsäure (PGA) wurden mit Gamma-Strahlen sterilisiert und in einem Vollmedium für eine Stunde in einen Inkubator gestellt. Die Fasermatrix KG119 betrifft als Flächenelement ein Vlies. Es wurde gemäß den in Tabelle 2 dargestellten Verfahrensparametern hergestellt. Der Zuschnitt wurde kreisförmig ausgestanzt (siehe Figur 3):

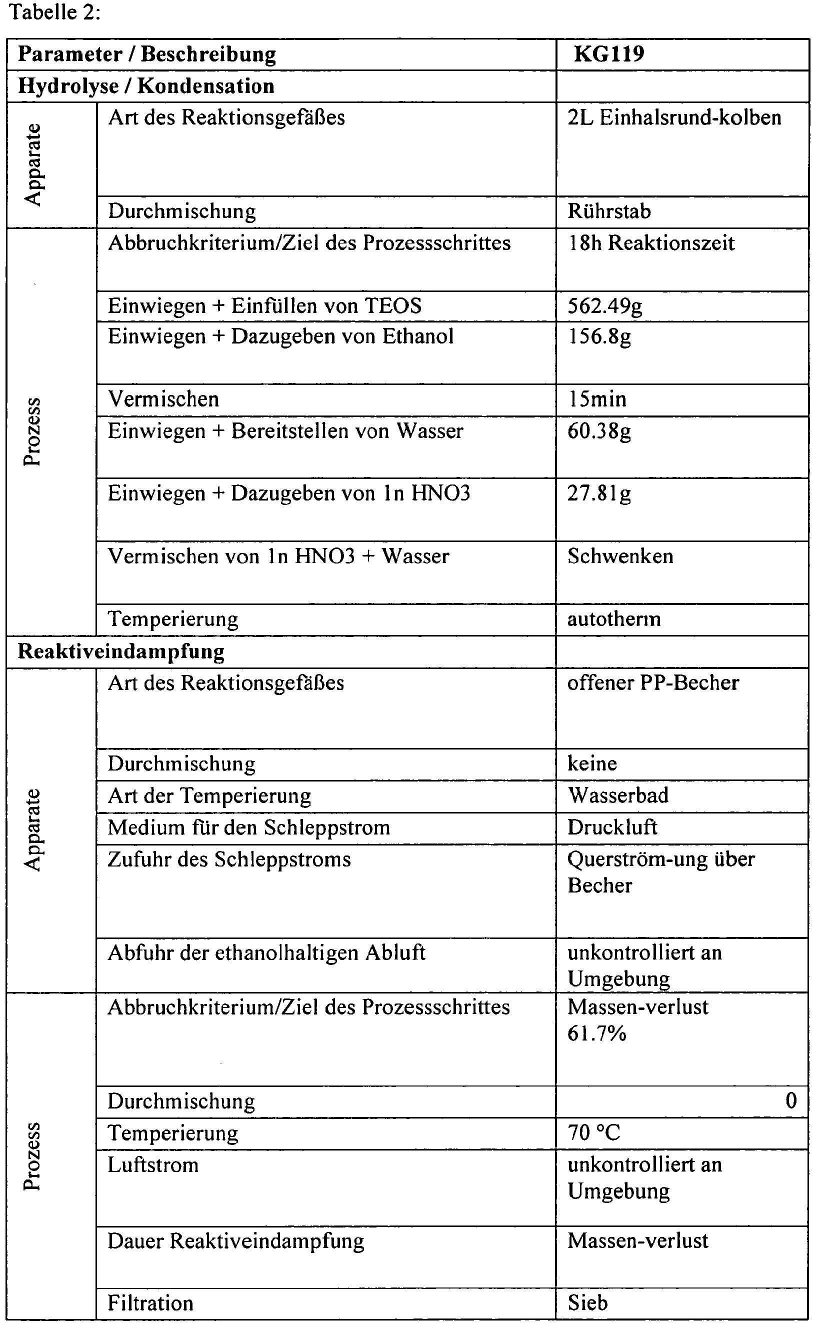

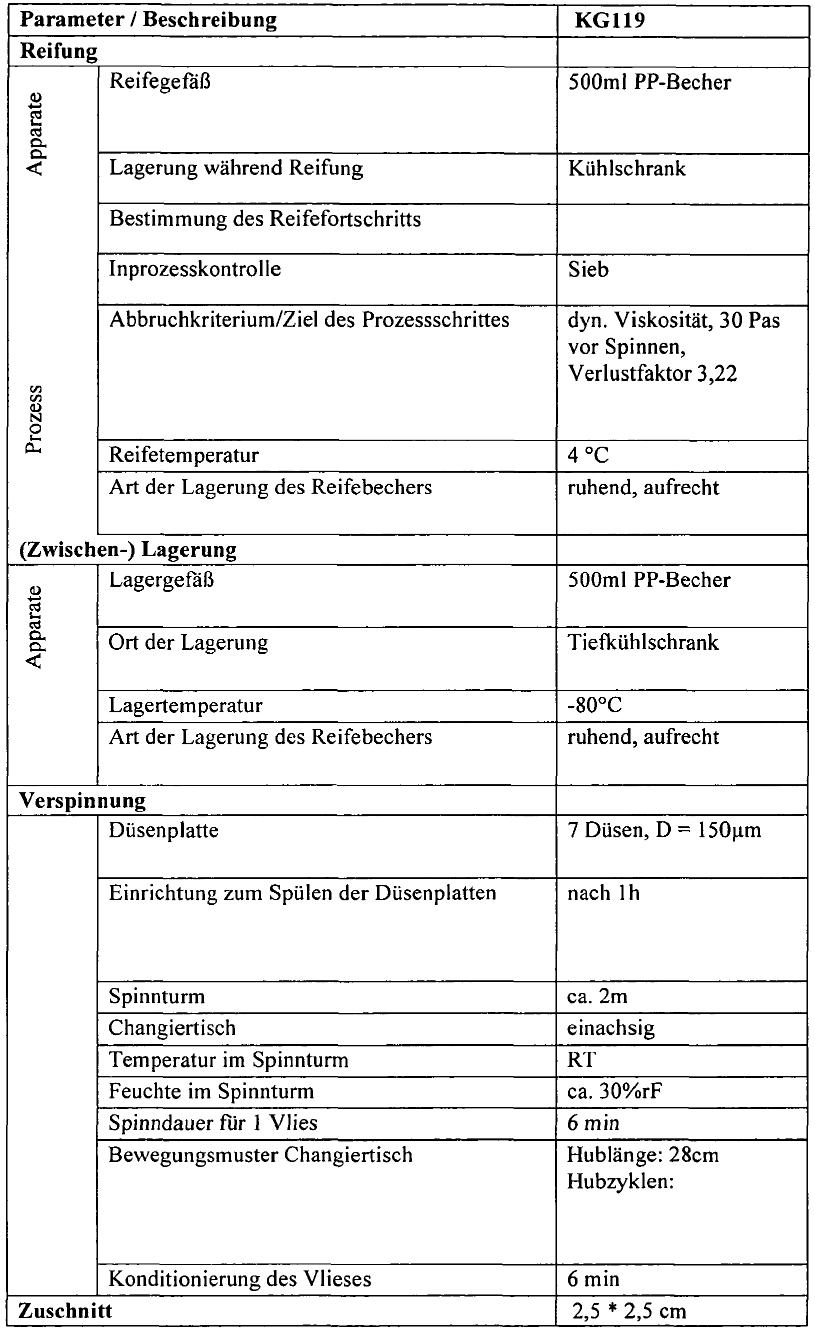

Vor der Zellbesiedlung wurde das Medium erneuert. Danach wurden humane dermale Fibroblastenzellen hinzugefügt. Die Zellkultur erfolgte in 24-Loch Falcon 351147 Plastikplatten. Das Medium wurde jeden Tag gewechselt. Zellbesiedlungsmedium war Gibco Dulbecco's Modified Eagle's Medium 42430-250 ergänzt mit 10% fötalem Kälberserum (FCS) und 100 Einheiten/mL Penizillin, 0,25 µg/mL Amphotericin B und 0,1 mg/mL Streptomycin als Antibiotika. Während des Wachstums der Zellen wurden nach dem anfänglichen Mediumswechsel 50 µg/mL Ascorbinsäure zum Medium dazugegeben. Weiter wurde es bei der steigenden Zellzahl notwendig, das Medium mit einem Natriumbikarbonat Lösungspuffer (7,5% Sigma) zu versetzen. Die Zellstandards (Kontrolle Zellen ohne Zellstützsubstanz) wurden in üblichen Gewebekulturschalen und Glassboden Iwaki Platten kultiviert.

Der Alamar Blue Assay wurde mit Reagentien von Serotec durchgeführt. Diese wurden auf 10% mit HBSS (Phenolfreiem) Puffer verdünnt, auf 37 °C eingestellt und sterilfiltriert. Die Zellstützsubstanzen mit den Zellen wurden in PBS gewaschen und dann von ihren ursprünglichen Platten entfernt und in Gewebekulturschalen und Glassboden Iwaki Platten platziert.

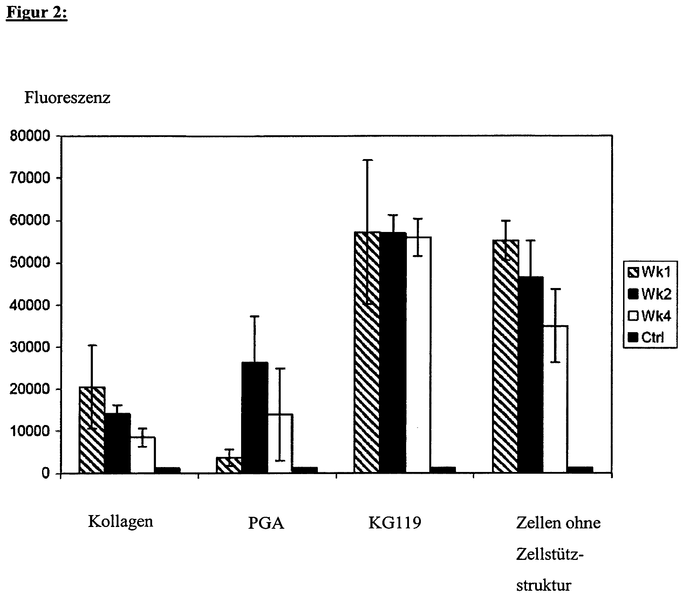

Die mit dem Alamar Blue Assay gemessene metabolische Aktivität ist eine Funktion der Zellzahl und der metabolischen Aktivität der einzelnen Zellen. Die Figur 2 vergleicht die Aktivität (dargestellt in Form eines Fluoreszenz Messwerts) der dermalen Fibroblasten auf den verschiedenen Matrices Kollagen, PGA und der Fasermatrix KG119 sowie Zellen ohne Stützgerüst (Kontrollkultur, Ctrl) bei einer Kulturdauer von einer Woche (Wk 1), 2 Wochen (Wk 2) und 4 Wochen (Wk 4).

Die primäre Adhesion der Zellen an KG119 ist stark und vergleichbar der von Kollagen. KG119 und Kollagen übertreffen PGA bezüglich Zelladhesion (Daten nicht gezeigt). Je länger die Zellen auf den Matrices wachsen, desto deutlicher zeigt sich die Überlegenheit der Fasermatrix KG119. Die Figur 2 zeigt, dass KG119 die anderen Zellstützstrukturen bzgl. metabolischer Aktivität der Zellen übertrifft. Die hohe metabolische Aktivität wird über den ganzen Messzeitraum (4 Wochen) beibehalten. Im Gegensatz dazu können Kollagen, PGA und Zellen ohne Zellstützstrukturen die metabolische Aktivität über diesen Zeitraum nicht aufrechterhalten. Einzig KG119 zeigt eine hohe Zelladhesion, Zellproliferation unter Beibehaltung der metabolischen Aktivität über den ganzen Zeitraum.

Figur 3 zeigt die Zellstützgerüste Kollagen, PGA und KG119 vor der Kultivierung mit human dermalen Fibroblastenzellen und nach 4 wöchiger Kulturdauer. Kollagen und PGA Zellstützgerüste ziehen sich zusammen und degradieren zu einem dichten Gewebeball. Einzig KG119 behält seine ursprüngliche Form. Innerhalb von KG119 hat sich eine dichte dermale Gewebemasse gebildet und die Fasern sind fest mit dem Gewebe verbunden.

### Beispiel 4

### Herstellung Kieselsol mit therapeutisch aktivem Wirkstoff

In einem temperierbaren Reaktionsgefäß wurden 5,4 Mol Tetraethoxysilan in Ethanol vorgelegt. Die Lösung wurde für ca. 15 min homogenisiert. Anschließend werden 56 g 1 N Salpetersäure verdünnt mit 121 g Wasser unter Rühren zugegeben. Die Mischung wurde für 18 h gerührt. Dabei verlief die Reaktion zunächst autotherm und wurde 2h nach Säurezugabe auf 37 °C temperiert.

Anschließend wurden 12 g Lidocain unter moderatem Rühren der Mischung zugeführt. Diese Mischung wurde in ein vorgeheiztes temperierbares Reaktionsgefäß überführt und bei ca. 64 °C und unter geringem Rühren über einen Zeitraum von 6 h auf ca 38,5% seiner ursprünglichen Masse eingedampft. Die Lösung war einphasig, enthielt keine Feststoffe und wies eine Viskosität von ca. 3 Pa s (Scherrate von 10 s⁻¹ bei 4 °C) auf.

Die Lösung wurde auf 4 °C abgekühlt und bei dieser Temperatur einer Reifung unterworfen.

### Beispiel 5

1124,98g TEOS werden im 21 Kolben mit 313,60 EtOH unter rühren 15 min gemischt. In einem 250ml Becherglas werden 120,76g H2 O, 55,62g 1n HNO₃ und 0,12g einer Nanosilberlösung (AgPURETM W 5%, rent a scientist GmbH) intensiv gerührt und optional 15 min im Ultraschall dispergiert und zum verdünntem TEOS gegeben. Dieser Ansatz wird standardmäßig insgesamt 18h gerührt (2h bei Raumtemperatur, anschließend 16h in einem 40°C warmen Wasserbad). Nach den 18 Stunden Reaktionsdauer sind einige schwarze Partikel im Sol zu sehen, nach einer 30 minütige Ultraschallbehandlung erhält man eine Sol mit nur noch ganz leichter Trübung. Dieses Sol wird im Reaktor bei 70°C reaktiveingedampft bis zu einem Gewichtsverlust von 61,7%. Bei 4°C reift das Sol zur Spinnmasse bis die gewünschten rheologischen Daten erreicht sind. Das Sol lässt sich gut verspinnen, die Wundauflagen haben eine ganz leichte Graufärbung im Vergleich zur undotierten Wundauflage.

## Patentansprüche

1. Verfahren zur Herstellung eines Kieselsol-Materials mit mindestens einem therapeutisch aktiven Wirkstoff umfassend die Schritte
a) Durchführen einer Hydrolyse-Kondensationsreaktion mit Tetraethoxysilan,
wobei die Hydrolyse-Kondensationsreaktion sauer katalysiert ist bei einem anfänglichen pH- Wert von 0 bis ≤ 7, gegebenenfalls in Gegenwart eines wasserlöslichen Lösungsmittels, und
die Hydrolyse-Kondensationsreaktion über mindestens 16 h bei einer Temperatur von 0 °C bis 80 °C durchgeführt wird,
b) Erzeugen einer einphasigen Lösung durch nachfolgendes Eindampfen,
wobei die einphasige Lösung eine Viskosität im Bereich von 0,5 bis 2 Pa•s bei einer Scherrate von 10 s⁻¹ bei 4 °C hat,
c) nachfolgendes Abkühlen dieser Lösung auf 2 °C bis 4 °C, und
d) Bildung eines homogenen Sols durch Unterwerfen dieser Lösung einer kinetisch kontrollierten Reifung bei einer Temperatur von 2 °C bis 4 °C,
wobei in einem oder mehreren der Schritte a) bis d) und vorzugsweise in einem oder mehreren der Schritte a) bis c) mindestens ein therapeutisch aktiver Wirkstoff dazugegeben wird.

2. Verfahren nach Anspruch 1, **dadurch gekennzeichnet, dass** für die saure Katalyse salpetersaures H₂O in einem molaren Verhältnis im Bereich 1:1,7 bis 1:1,9, bevorzugt im Bereich von 1:1,7 bis 1:1,8, eingesetzt wird und die Hydrolyse-Kondensationsreaktion mindestens 16 h, bevorzugt 18 h, und zwischen 20 °C und 60 °C, besonders bevorzugt bei Raumtemperatur (20 °C bis 25 °C), durchgeführt wird.

3. Verfahren nach Anspruch 1 oder 2, **dadurch gekennzeichnet, dass** die Hydrolyse-Kondensationsreaktion in Schritt a) bei 20 °C bis 60 °C, bevorzugt 20 °C bis 50 °C, besonders bevorzugt bei Raumtemperatur (20 °C bis 25 °C) über einen Zeitraum von mindestens 16 h bis zu 4 Wochen, bevorzugt 18 h bis 4 Wochen, besonders bevorzugt 24 h bis 18 Tage, ganz besonders bevorzugt zwischen 3 bis 8 Tage, durchgeführt wird.

4. Verfahren nach einem der vorherigen Ansprüche, **dadurch gekennzeichnet, dass** der Schritt b) in einer geschlossenen Apparatur bei einer Reaktionstemperatur von etwa 30 °C bis etwa 90 °C abläuft.

5. Verfahren nach einem der vorhergehenden Ansprüche, **dadurch gekennzeichnet, dass** die Lösung in Schritt c) auf 4 °C abgekühlt wird.

6. Verfahren nach einem der vorhergehenden Ansprüche, **dadurch gekennzeichnet, dass** die Reifung in Schritt d) bei 4 °C erfolgt.

7. Verfahren nach einem der vorhergehenden Ansprüche, **dadurch gekennzeichnet, dass** in Schritt d) die Reifung bis zu einer Viskosität des Sols von 30 bis 100 Pa•s bei einer Scherrate von 10 s¹ bei 4 °C und einem Verlustfaktor von 2 bis 5 (bei 4 °C, 10 s⁻¹, 1 % Deformation) durchgeführt wird.

8. Verfahren nach einem der vorhergehenden Ansprüche, umfassend mindestens einen weiteren Schritt, wobei aus dem in Schritt d) gewonnene Sol ein biologisch resorbierbare(s) und/oder bioaktive(s) Pulver, ein Monolith und/oder eine Beschichtung hergestellt wird.

9. Verfahren nach einem der Ansprüche 1 bis 8, **dadurch gekennzeichnet, dass** in Schritt b) gleichzeitig mit dem Eindampfen das Reaktionsgemisch schonend durchmischt wird

10. Verfahren nach einem der Ansprüche 1 bis 7, umfassend einen weiteren Schritt e), wobei das in Schritt d) gewonnene Sol zu einem biologisch degradierbaren und/oder biologisch resorbierbaren Fasermaterial in einem Spinnprozess versponnen wird.

## Claims

1. Process for producing a silica sol material containing at least one therapeutically active ingredient, comprising the steps
a) carrying out a hydrolysis-condensation reaction with tetraethoxysilane,
wherein the hydrolysis-condensation reaction is acid-catalysed at an initial pH of 0 to ≤ 7, optionally in the presence of a watersoluble solvent, and
the hydrolysis-condensation reaction is carried out for at least 16 h at a temperature of 0°C to 80°C,
b) generating a single-phase solution by subsequent evaporation,
wherein the single-phase solution has a viscosity within the range from 0.5 to 2 Pa · s at a shear rate of 10 s⁻¹ at 4°C,
c) subsequently cooling said solution to 2°C to 4°C, and
d) forming a homogeneous sol by subjecting said solution to a kinetically controlled maturation at a temperature of 2°C to 4°C,
wherein at least one therapeutically active ingredient is added in one or more of steps a) to d) and preferably in one or more of steps a) to c) .

2. Process according to Claim 1, **characterized in that** nitric acid-acidified H₂O is used in a molar ratio within the range from 1:1.7 to 1:1.9, preferably within the range from 1:1.7 to 1:1.8, for the acidic catalysis and the hydrolysis-condensation reaction is carried out for at least 16 h, preferably 18 h, and between 20°C and 60°C, particularly preferably at room temperature (20°C to 25°C).

3. Process according to Claim 1 or 2, **characterized in that** the hydrolysis-condensation reaction in step a) is carried out at 20°C to 60°C, preferably 20°C to 50°C, particularly preferably at room temperature (20°C to 25°C), for a period of at least 16 h up to 4 weeks, preferably 18 h to 4 weeks, particularly preferably 24 h to 18 days, very particularly preferably between 3 to 8 days.

4. Process according to any of the preceding claims, **characterized in that** step b) proceeds in a closed apparatus at a reaction temperature of about 30°C to about 90°C.

5. Process according to any of the preceding claims, **characterized in that** the solution in step c) is cooled to 4°C.

6. Process according to any of the preceding claims, **characterized in that** the maturation in step d) is done at 4°C.

7. Process according to any of the preceding claims, **characterized in that**, in step d), the maturation is carried out up to a sol viscosity of 30 to 100 Pa · s at a shear rate of 10 s⁻¹ at 4°C and a loss factor of 2 to 5 (at 4°C, 10 s⁻¹, 1% deformation).

8. Process according to any of the preceding claims, comprising at least one further step, wherein a bioabsorbable and/or bioactive powder, a monolith and/or a coating is produced from the sol obtained in step d).

9. Process according to any of Claims 1 to 8, **characterized in that**, in step b), the reaction mixture is gently mixed at the same time as the evaporation.

10. Process according to any of Claims 1 to 7, comprising a further step e), wherein the sol obtained in step d) is spun in a spinning process to form a biodegradable and/or bioabsorbable fibre material.

## Revendications

1. Procédé pour la préparation d'un matériau de type sol silicique présentant au moins une substance thérapeutiquement active, comprenant les étapes
a) réalisation d'une réaction d'hydrolyse-condensation avec un tétraéthoxysilane,
la réaction d'hydrolyse-condensation étant catalysée par voie acide à un pH de départ de 0 à ≤ 7, le cas échéant en présence d'un solvant soluble dans l'eau, et
la réaction d'hydrolyse-condensation étant réalisée pendant au moins de 16 h à une température de 0°C à 80°C,
b) obtention d'une solution à une phase par évaporation consécutive, la solution à une phase présentant une viscosité dans la plage de 0,5 à 2 Pa.s à une vitesse de cisaillement de 10 s⁻¹ à 4°C,
c) refroidissement consécutif de cette solution à 2°C jusqu'à 4°C et
d) formation d'un sol homogène par soumission de cette solution à une maturation cinétiquement contrôlée à une température de 2°C jusqu'à 4°C,
au moins une substance thérapeutiquement active étant ajoutée dans une ou plusieurs des étapes a) à d) et de préférence dans une ou plusieurs des étapes a) à c).

2. Procédé selon la revendication 1, **caractérisé en ce qu'**on utilise, pour la catalyse acide, H₂O acide nitrique dans un rapport molaire dans la plage de 1:1,7 à 1:1,9, de préférence dans la plage de 1:1,7 à 1:1,8, et on réalise la réaction d'hydrolyse-condensation pendant au moins 16 h, de préférence 18 h, et entre 20°C et 60°C, de manière particulièrement préférée à température ambiante (20°C à 25°C).

3. Procédé selon la revendication 1 ou 2, **caractérisé en ce que** la réaction d'hydrolyse-condensation dans l'étape a) est réalisée à 20°C jusqu'à 60°C, de préférence à 20°C jusqu'à 50°C, de manière particulièrement préférée à température ambiante (20°C à 25°C) sur une période de temps d'au moins 16 h jusqu'à 4 semaines, de préférence de 18 h à 4 semaines, de manière particulièrement préférée de 24 h à 18 jours, de manière tout particulièrement préférée de 3 à 8 jours.

4. Procédé selon l'une quelconque des revendications précédentes, **caractérisé en ce que** l'étape b) se déroule dans un appareillage fermé à une température de réaction d'environ 30°C à environ 90°C.

5. Procédé selon l'une quelconque des revendications précédentes, **caractérisé en ce que** la solution dans l'étape c) est refroidie à 4°C.

6. Procédé selon l'une quelconque des revendications précédentes, **caractérisé en ce que** la maturation dans l'étape d) a lieu à 4°C.

7. Procédé selon l'une quelconque des revendications précédentes, **caractérisé en ce que**, dans l'étape d), la maturation est réalisée jusqu'à une viscosité du sol de 30 à 100 Pa.s à une vitesse de cisaillement de 10 s⁻¹ à 4°C et à un facteur de perte de 2 à 5 (à 4°C, 10 s⁻¹, déformation de 1%) .

8. Procédé selon l'une quelconque des revendications précédentes, comprenant au moins une autre étape dans laquelle une poudre biologiquement résorbable(s) et/ou bioacti(f)(ve)(s), un monolithe et/ou un revêtement est/sont préparé(e)(s) à partir du sol obtenu dans l'étape d).

9. Procédé selon l'une quelconque des revendications 1 à 8, **caractérisé en ce que**, dans l'étape b), le mélange réactionnel est mélangé de manière ménagée tout en l'évaporant.

10. Procédé selon l'une quelconque des revendications 1 à 7, comprenant une autre étape e) dans laquelle le sol obtenu dans l'étape d) est filé dans un procédé de filage en un matériau fibreux biologiquement dégradable et/ou biologiquement résorbable.
